# EUROPEAN PATENT SPECIFICATION

(11) **EP 1 626 715 B1**
(45) Date of publication and mention of the grant of the patent: **10.09.2008**
(21) Application number: 04731575.9
(22) Date of filing: 07.05.2004
(51) Int. Cl.: A61K 31/404, A61K 31/4045, A61K 31/454, A61K 31/4535, A61K 31/496, A61K 31/5377, A61P 3/04, A61P 3/10

(54) **USE OF SULPHONAMIDE DERIVATIVES FOR THE MANUFACTURE OF A MEDICAMENT FOR THE PROPHYLAXIS AND/OR TREATMENT OF DISORDERS OF FOOD INGESTION**
VERWENDUNG VON SULFONAMID-DERIVATE ZUR HERSTELLUNG EINES MEDIKAMENTS FÜR DIE VORBEUGUNG ODER BEHANDLUNG VON ESSSTÖRUNGEN
UTILISATION DE DERIVES SULFAMIDES POUR LA FABRICATION D'UN MEDICAMENT POUR LA PROPHYLAXIE ET/OU LE TRAITEMENT DE TROUBLES D'INGESTION DES ALIMENTS

(30) Priority: 09.05.2003 ES 200301077; 28.07.2003 ES 200301782
(43) Date of publication of application: 22.02.2006
(73) Proprietor: LABORATORIOS DEL DR. ESTEVE, S.A., 08041 Barcelona (ES)
(72) Inventor: MERCE, VIDAL, Ramon, E-Barcelona (ES); ANDALUZ, MATARÓ, Blas, E-Tordera (ES); FRIGOLA, CONSTANSA, Jordi, E-Sant Just Desvern (ES)
(74) Representative: Carpintero Lopez, Francisco
(86) International application number: PCT/EP2004/004882
(87) International publication number: WO 2004/098588

(56) References cited:
- EP-A- 0 733 628
- WO-A-02/102774
- WO-A-03/042175
- US-A- 5 708 008

## Description

The present invention relates to the use of sulphonamide derivatives of general formula (I), 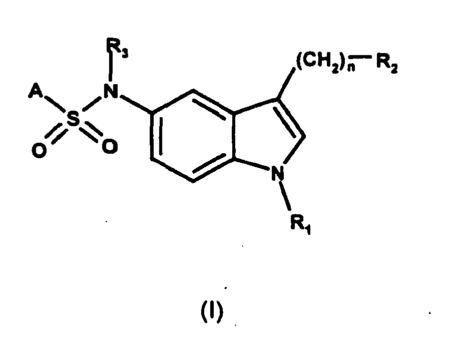 optionally in form of one of their stereoisomers, preferably enantiomers or diastereomers, their racemates or in form of a mixture of at least two of their stereoisomers, preferably enantiomers or diastereomers, in any mixing ratio, or corresponding physiologically acceptable salts or corresponding solvates, for the manufacture of a medicament for the prophylaxis and/or treatment of disorders of food ingestion.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacal. Exp. Ther. , 1995, 274, 173; A.J. Sleight, et al., Behav. Brain Res. , 1996, 73, 245; T.A. Branchek, et al., nnu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379].

Moreover, it has been shown that the 5-HT₆ receptor also plays a role in food ingestion [Neuropharmacology, 41, 2001, 210-219].
Food ingestion disorders, particularly obesity, are a serious, fast growing threat to the health of humans of all age groups, since they increase the risk of developing other serious, even life-threatening diseases such as diabetes or coronary diseases.

Thus, the object of the present invention was to provide medicaments that comprise compounds with 5-HT₆ receptor affinity and which are suitable for the prophylaxis and/or treatment of food-ingestion related disorders.

It has been found that the sulphonamide derivatives of general formula (I) given below show affinity for the 5-HT₆-receptor. These compounds are therefore also suitable for the manufacture of a medicament for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity.

The sulphonamide derivatives of general formula (I) of the invention are disclosed in document WO 03/042175 A as compounds useful for the prevention or treatment of several CNS diseases.

On the other hand, other compounds with a different structural formula have been described for the prevention or treatment of food ingestion disorders. In documents EP 0733628 A1 and US 5,708,008 similar sulphonamide derivatives but with different substitution in the indole ring are disclosed as compounds useful for the treatment of migraine and associated disorders such as bulimia and anorexia nervosa, among many others. Also, indole derivatives with a different core structure are disclosed in document WO 02/102774 as compounds useful for the treatment of CNS disorders, gastrointestinal disorders and obesity.

Therefore, the use of the sulphonamide derivatives of general formula (I) of the invention for the manufacture of a medicament for the prophylaxis and/or treatment of disorders of food ingestion is not disclosed or suggested in the prior art.

Thus, one aspect of the present invention is the use of at least one sulphonamide derivative of general formula (I), 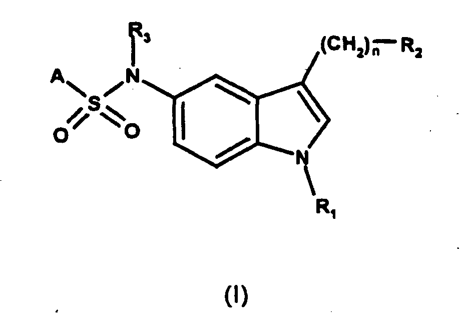 wherein
R¹ represents hydrogen, an optionally at least mono-substituted, linear or branched alkyl radical, an optionally at least mono-substituted phenyl radical or an optionally at least mono-substituted benzyl radical,
R² represents a -NR⁴R⁵ moiety
R³ represents hydrogen or an optionally at least mono-substituted, linear or branched alkyl radical,
R⁴ and R⁵, identical or different, represent hydrogen or a linear or branched C₁-C₄ alkyl radical, or
R⁴ and R⁵ together with the bridging nitrogen atom form a moiety selected from the group consisting of 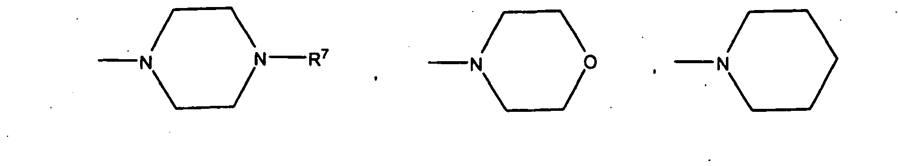 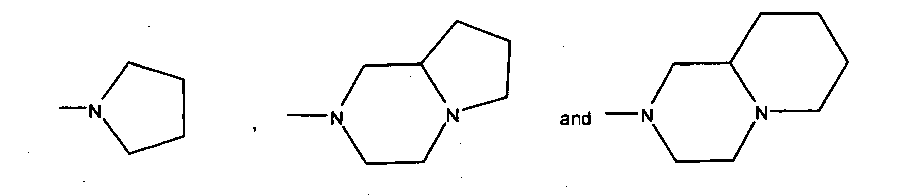 wherein R⁷ represents hydrogen, a linear or branched C₁-C₄-alkyl radical or a benzyl radical, preferably hydrogen or a C₁-C₂ alkyl radical,

A represents an optionally at least mono-substituted mono- or polycyclic aromatic ringsystem, which may be bonded via an optionally at least mono-substituted alkylene-, alkenylene- or alkynylene group and/or may contain at least one heteroatom as a ring member in one or more of its rings,
n represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate,
for the manufacture of a medicament for the prophylaxis and/or treatment of a food ingestion disorder.

If one or more of the residues R¹, R³, R⁴ and R⁵ represents an alkyl radical, which is substituted with one or more substituents, unless defined otherwise, each of the substituents may preferably be selected from the group consisting of hydroxy, fluorine, chlorine, bromine and trifluoromethyl.

If R¹ represents a phenyl radical or a benzyl radical, which is substituted with one or more substituents, unless defined otherwise, each of the substituents may preferably be selected from the group consisting of hydroxy, fluorine, chlorine, bromine, branched or unbranched C₁-C₄-alkyl, branched or unbranched C₁-C₄-alkoxy, branched or unbranched C₁-C₄-perfluoroalkyl and branched or unbranched C₁-C₄-perfluoroalkoxy.

If A represents a mono- or polycyclic aromatic ringsystem, which is substituted with one or more substituents, and which may be bonded via an optionally at least mono-substituted alkylene-, alkenylene- or alkynylene group and/or may contain at least one heteroatom as a ring member, unless otherwise defined, each of the substituents, may preferably be selected from the group consisting of hydroxy, halogen, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy, an optionally at least mono-substituted phenyl radical, an optionally at least mono-substituted phenoxy radical and 5-or 6 membered heteroaryl, preferably from the group consisting of halogen, branched or unbranched C₁-C₄-alkyl, an optionally at least mono-substituted phenyl radical, an optionally at least mono-substituted phenoxy radical and 5- or 6-membered heteroaryl, more preferably from the group consisting of fluorine, chlorine, branched or unbranched C₁-C₄-alkyl, an optionally at least mono-substituted phenyl radical, an optionally at least mono-substituted phenoxy radical and 5- or 6-membered heteroaryl selected from the group consisting of furyl, thienyl and pyridyl. If one or more of the rings of the mono- or polycyclic aromatic ringsystem contains one or more heteroatoms, these heteroatoms - like the heteroatoms of the afore mentioned 5-or 6 membered heteroaryl radical - may preferably be selected from the group consisting of oxygen, sulphur and nitrogen. If the afore mentioned phenyl radical is itself substituted with one or more substituents, each of the substituents may preferably be selected from the group consisting of fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR⁸R⁹-moiety, wherein R⁸ and R⁹, identical or different, represent hydrogen or linear or branched C₁-C₄-alkyl.

If the afore mentioned alkylene-, alkenylene- or alkynylene group is substituted with one or more substituents, each of the substituents may preferably be selected from the group consisting of hydroxy, halogen, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy or an optionally at least mono-substituted phenyl radical. If said phenyl radical is itself substituted by one or more substituents, each of the substituents may preferably be selected from the group consisting of fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR⁸R⁹-moiety, wherein R⁸ and R⁹, identical or different, represent hydrogen or linear or branched C₁-C₄-alkyl.

Preferably used are sulphonamide derivatives of general formula (I), wherein R¹ represents hydrogen, an optionally at least mono-substituted, linear or branched C₁₋₄-alkyl radical, an optionally at least mono-substituted phenyl radical or an optionally at least mono-substituted benzyl radical, preferably hydrogen, a linear or branched C₁₋₄-alkyl radical or a benzyl radical, more preferably hydrogen, and R² to R⁵, A and n are as defined above, optionally in form of one of the stereoisomers, preferably enantiomers or diastereomers, the racemate or in form of a mixture of at least two of the stereoisomers, preferably enantiomers or diastereomers, in any mixing ratio, or a corresponding physiologically acceptable salt or a corresponding solvate.

Also preferred is the use of sulphonamide derivatives of general formula (I), wherein R³ represents hydrogen or an optionally at least mono-substituted, linear or branched C₁-C₄-alkyl radical, preferably hydrogen or a linear or branched C₁-C₄-alkyl radical, more preferably hydrogen or a C₁-C₂ alkyl radical, and R¹, R² R⁴, R⁵, A and n are as defined above, optionally in form of one of the stereoisomers, preferably enantiomers or diastereomers, the racemate or in form of a mixture of at least two of the stereoisomers, preferably enantiomers or diastereomers, in any mixing ratio, or a corresponding physiologically acceptable salt or a corresponding solvate.

Particularly preferred is the use of sulphonamide derivatives of general formula (I), wherein R⁴ and R⁵, identical or different, represent hydrogen or a linear or branched C₁-C₄-alkyl radical, preferably a linear or branched C₁-C₄-alkyl radical, or
R⁴ and R⁵ together with the bridging nitrogen atom form a moiety selected from the group consisting of 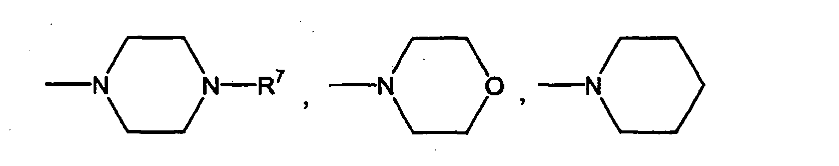 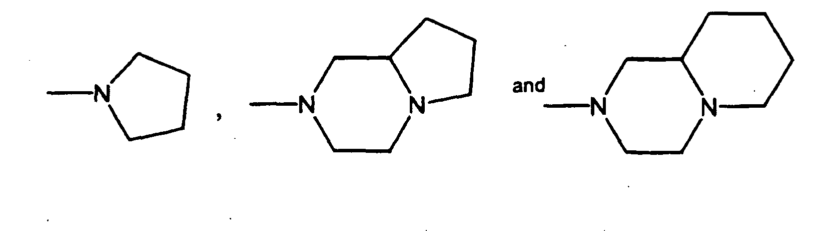 wherein R⁷ represents hydrogen, a linear or branched C₁-C₄-alkyl radical or a benzyl radical, preferably hydrogen or a C₁-C₂ alkyl radical, and R¹-R³, A and n are as defined above, optionally in form of one of the stereoisomers, preferably enantiomers or diastereomers, the racemate or in form of a mixture of at least two of the stereoisomers, preferably enantiomers or diastereomers, in any mixing ratio, or a corresponding physiologically acceptable salt or a corresponding solvate.

Moreover, the use of sulphonamide derivatives of general formula (I) is preferred, wherein A represents an optionally at least mono-substituted mono- or bicyclic aromatic ringsystem, wherein the ring(s) is/are 5- or 6-membered, which may be bonded via a an optionally at least mono-substituted C₁-C₄-alkylene group, an optionally at least mono-substituted C₂-C₄-alkenylene or an optionally at least mono-substituted C₂-C₄-alkinylene group and/or may contain at least one heteroatom as a ring member, preferably an optionally at least mono-substituted mono- or bicyclic aromatic ringsystem, wherein the ring(s) is/are 5- or 6-membered and wherein one or both of the rings contain(s) at least one heteroatom, or a moiety selected from the group consisting of 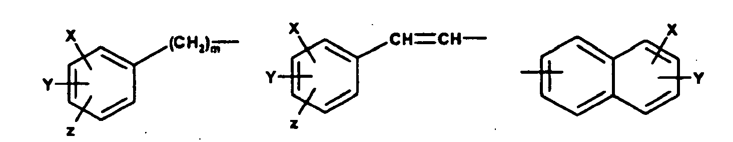 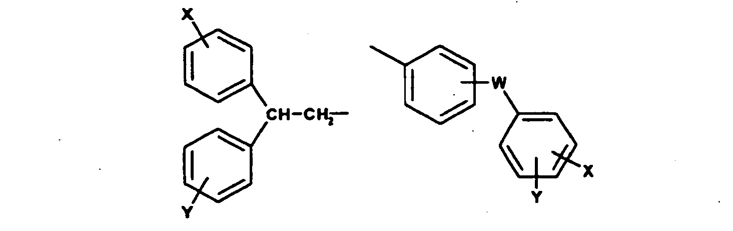 wherein X, Y, Z are each independently selected from the group consisting of hydrogen, fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR⁸R⁹-moiety, wherein R⁸ and R⁹, identical or different, represent hydrogen or linear or branched C₁-C₄-alkyl,
W represents a single chemical bond between the two rings, a CH₂-group, O, S or a NR¹⁰-moiety, wherein R¹⁰ is hydrogen or linear or branched C₁-C₄-alkyl and
m is 0, 1, 2, 3 or 4.
and R¹-R⁵ and n are as defined above, optionally in form of one of the stereoisomers, preferably enantiomers or diastereomers, the racemate or in form of a mixture of at least two of the stereoisomers, preferably enantiomers or diastereomers, in any mixing ratio, or a corresponding physiologically acceptable salt or a corresponding solvate.

Most preferred is the use of one or more sulphonamide derivatives selected from the group consisting of:
[1] N-[3-(2-diethylaminoethyl)-1H-indol-5-yl]-5-chloro-3-methylbenzo[b]thiophene-2-sulphonamide,
[2] N-[3-(2-diethylaminoethyl)-1H-indol-5-yl]naphthalene-1-sulphonamide,
[3] Hydrochloride N-[3-(2-diethylaminoethyl)-1*H*-indol-5-yl]naphthalene-1-sulphonamide,
[4] N-[3-(2-diethylaminoethyl)-1*H*-indol-5-yl]-3,5-dichlorobenzenesulphonamide,
[5] N-[3-(2-diethylaminoethyl)-1*H*-indol-5-yl]-4-phenylbenzenesulphonamide,
[6] N-[3-(2-diethylaminoethyl)-1*H*-indol-5-yl]-5-chlorothiophene-2-sulphonamide,
[7] N-[3-(2-dimethylaminoethyl)-1*H*-indol-5-yl]-5-chloro-3-methylbenzo[b]thiophene-2-sulphonamide,
[8] N-[3-(2-dimethylaminoethyl)-1*H*-indol-5-yl]naphthalene-1-sulphonamide,
[9] N-[3-(2-dimethylamino-ethyl)-1*H*-indol-5-yl]-6-chloroimidazo[2,1-b]thiazol-5-sulphonamide,
[17] N-[3-(2-diethylaminoethyl)-1*H*-indol-5-yl]naphthalene-2-sulphonamide,
[19] N-[3-(4-methylpiperazin-1-yl)methyl-1*H*-indol-5-yl]-5-chloro-3-methylbenzo[b]thiophene-2-sulphonamide,
[20] N-[3-(2-dimethylaminoethyl)-1*H*-indol-5-yl]-5-(2-pyridil)thiophene-2-sulphonamide,
[21] N-[3-(2-dimethylaminoethyl)-1*H*-indol-5-yl]-2,1,3-benzothiadiazol-4-sulphonamide,
[22] N-[3-(2-dimethylaminoethyl)-1*H*-indol-5-yl]quinoline-8-sulphonamide,
[23] N-[3-(2-dimethylaminoethyl)-1*H*-indol-5-yl]-5-chloronaphthalene-2-sulphonamide,
[24] N-[3-(2-dimethylaminoethyl)-1*H*-indol-5-yl]-4-phenoxybenzenesulphonamide,
[25] N-[3-(2-dimethylaminoethyl)-1*H*-indol-5-yl]-4-phenylbenzenesulphonamide,
[26] N-[3-(2-diethylaminoethyl)-1*H*-indol-5-yl]-N-ethyl-naphthalene-2-sulphonamide,
[27] N-{3-[2-(morpholin-4-yl)ethyl]-1H-indol-5-yl}-5-chloro-3-methylbenzo[b]thiophene-2-sulphonamide,
[28] N-{3-[2-(morpholin-4-yl)ethyl]-1H-indol-5-yl}naphthalene-1-sulphonamide,
[29] N-[3-(2-diethylaminoethyl)-1H-indol-5-yl]naphthalene-2-sulphonamide,
[30] N-[3-dimethylaminomethyl-1H-indol-5-yl]-5-chloro-3-methylbenzo[b]thiophene-2-sulphonamide,
[31] N-[3-(2-dipropylaminoethyl)-1H-indol-5-yl]naphthalene-1-sulphonamide,
[32] N-[3-(2-dipropylaminoethyl)-1H-indol-5-yl]-5-chloro-3-methylbenzo[b]thiophene-2-sulphonamide,
[33] N-[3-(2-dibutylaminoethyl)-1H-indol-5-yl]-5-chloro-3-methylbenzo[b]thiophene-2-sulphonamide,
[34] N-[3-(2-dibutylaminoethyl)-1H-indol-5-yl]naphthalene-1-sulphonamide,
[35] N-[3-(2-diethylaminoethyl)-1H-indol-5-yl]-5-chloronaphthalene-1-sulphonamide,
[36] N-[3-(2-diethylaminoethyl)-1H-indol-5-yl]-trans-β-styrenesulphonamide,
[37] N-[3-(4-methylpiperazin-1-yl)methyl-1*H*-indol-5-yl]-trans-β-, styrenesulphonamide,
[39] N-[3-(2-diethylaminoethyl)-1*H*-indol-5-yl]-6-chloroimidazo[2,1-b]thiazol-5-sulphonamide,
[40] N-{3-[2-(morpholin-4-yl)ethyl]-1H-indol-5-yl}naphthalene-2-sulphonamide,
[41] N-[3-(4-methylpiperazin-1-yl)methyl-1*H*-indol-5-yl]-α-toluenesulphonamide,
[42] N-[3-(3-diethylaminopmpyl)-1H-indol-5-yl]naphthalene-2-sulphonamide,
[43] N-[3-(3-diethylaminopropyl)-1H-indol-5-yl]-5-chloro-3-methylbenzo[b]thiophene-2-sulphonamide,
[44] N-{3-[2-(pyrrolidin-1-yl)ethyl]-1H-indol-5-yl}-5-chloro-3-methylbenzo[b]thiophene-2-sulphonamide,
[45] N-{3-[2-(pyrrolidin-1-yl)ethyl]-1H-indol-5-yl}naphthalene-1-sulphonamide,
[46] N-{3-[2-(pyrrolidin-1-yl)ethyl]-1H-indol-5-yl}naphthalene-2-sulphonamide,
[47] N-[3-(2-dipropylaminoethyl)-1H-indol-5-yl]naphthalene-2-sulphonamide,
[48] N-[3-(2-dimethylaminoethyl)-1H-indol-5-yl]-5-chloronaphthalene-1-sulphonamide,
[49] N-[3-(2-dimethylaminoethyl)-1H-indol-5-yl]naphthalene-2-sulphonamide,
[50] N-{3-[2-(morpholin-4-yl)ethyl]-1H-indol-5-yl}quinoline-8-sulphonamide, and
[51] N-{3-[2-(morpholin-4-yl)ethyl]-1H-indol-5-yl}-4-phenylbenzenesulphonamide,

The sulphonamide derivatives of general formula (I), wherein R₁, R₂, R₃, n and A have the above defined meaning, may preferably be prepared according to the following methods:

### METHOD A:

At least one compound of general formula (II), 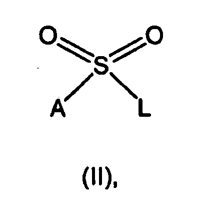 wherein A has the meaning as defined above and L is a suitable leaving group, preferably a halogen atom, particularly preferably chlorine; is reacted with at least one substituted 5-aminoindol of general formula (III) 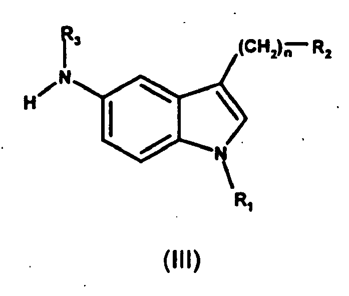 wherein R₁, R₂, R₃ and n have the meaning as defined above, or a suitably protected derivative thereof, and, if present, the protective groups are removed, in order to obtain the corresponding sulphonamide derivative of general formula (I), which may be purified and/or may be isolated by conventional methods known to those skilled in the art.

The reaction between the compounds of general formulas (II) and (III) is usually carried out in the presence of an organic reaction medium, such as an dialkyl ether, particularly diethyl ether, or a cyclic ether, particularly tetrahydrofurane or dioxane, a halogenated organic hydrocarbon, particularly methylene chloride or chloroform, an alcohol, particularly methanol or ethanol, an aprotic dipolar solvent, particularly acetonitrile, pyridine or dimethylformamide, or any other suitable reaction medium. Mixtures of at least two of the above mentioned classes of compounds or of at least two compounds of one class may, of course, also be used.

The reaction is preferably carried out in the presence of a suitable base, e.g. an inorganic base such as hydroxides and/or carbonates of alkali metals, or an organic base, particularly triethylamine or pyridine.

The most suitable reaction temperatures range from 0° C to ambient temperature, i.e. approximately 25 °C, and the reaction time is preferably from 5 minutes to 24 hours.

The resulting sulphonamide derivative of general formula (I) may be purified and/or isolated according to conventional methods known to those skilled in the art.

Preferably the sulphonamide derivatives of general formula (I) can be isolated by evaporating the reaction medium, adding water and eventually adjusting the pH so that it is obtained as a solid that can be isolated by filtration; or it can be extracted by a solvent immiscible with water, such as chloroform, and purified by chromatography or recrystallisation from a suitable solvent.

The compounds of general formula (II) are commercially available or can be prepared according to standard methods known to those skilled in the art, e.g. by methods analogous to those described in the literature [E.E. Gilbert, Synthesis, 1969, 1, 3]. The compounds of general formula (III) may also be prepared according to standard methods known to those skilled in the art, e.g. by methods analogous to those described in the literature [J.E. Macor, R. Post and K. Ryan, Synt Comm., 1993, 23, 1, 65-72.; J. Guillaume, C. Dumont, J. Laurent and N. Nédélec, Eur. J. Med. Chem., 1987, 22, 33-43; M.L. Saccarello, R. Stradi, Synthesis, 1979, 727].

### METHOD B

The sulphonamide derivatives of general formula (I), wherein R₁, R₂, n and A are as defined above and R₃ represents an optionally at least mono-substituted, linear or branched C₁-C₄ alkyl radical, may also be prepared by alkylation of a corresponding sulphonamide derivative of general formula (I), wherein R₁, R₂, n and A are as defined above and R₃ represents a hydrogen atom, with an alkyl halogenide or a dialkyl sulphate.

The alkylation reaction is preferably carried out in the presence of a suitable base, such as hydroxides and/or carbonates of alkali metals, metal hydrides, alkoxides such as sodium methoxide or potassium tert-butoxide, organometallic compounds such as butyl lithium or tert.-butyl lithium, in the presence of an organic reaction medium, such as dialkyl ether, particularly diethyl ether, or a cyclic ether, particularly tetrahydrofurane or dioxane, a hydrocarbon, particularly toluene, an alcohol, particularly methanol or ethanol, an aprotic dipolar solvent, particularly acetonitrile, pyridine or dimethylformamide, or any other suitable reaction medium. Mixtures of at least two of the above mentioned classes of compounds and/or of at least two compounds of one class may, of course, also be used.

The most suitable reaction temperatures range from 0° C to the boiling point of the reaction medium, and reaction times preferably range from 1 to 24 hours.

The resulting sulphonamide derivative of general formula (I) can preferably be isolated by filtration, concentrating the filtrate at reduced pressure, adding water and eventually adjusting the pH so that it is obtained as a solid that can be isolated by filtration, or it can be extracted with a solvent immiscible in water such as chloroform and purified by chromatography or recrystallisation from a suitable solvent.

### METHOD E

The pharmacologically acceptable salts of compounds with the general formula (I) can be prepared by conventional methods known to those skilled in the art, preferably by reaction with a mineral acid, such as hydrochloric, hydrobromic, phosphoric, sulphuric, nitric acids or with organic acids such as citric, maleic, fumaric, tartaric acids or their derivatives, *p*-toluensulphonic acid, methansulphonic acid, etc., in a suitable solvent such as methanol, ethanol, diethyl ether, ethyl acetate, acetonitrile or acetone and obtained with the usual techniques of precipitation or crystallisation of the corresponding salts.

Preferred physiologically acceptable salts of the sulphonamide derivatives of general formula (I) are the additions salts of mineral acids, such as hydrochloric acid, hydrobromic acid, phosphoric acid, sulphuric acid, nitric acid, and of organic acids, such as citric acid, maleic acid, tartaric acid or derivatives thereof, p-toluenesulphonic acid, methansulphonic acid, camphorsulphonic acid, etc.

The physiologically acceptable solvates, particularly hydrates, of the sulphonamide derivatives of general formula (I) or of the corresponding physiologically acceptable salts may be prepared by conventional methods known to those skilled in the art.

During one of the synthesis sequences described above, or in the preparation of suitable reactands used it may be necessary and/or desirable to protect sensitive or reactive groups in some of the molecules employed. This can be performed by means of conventional protective groups such as those described in the literature [Protective groups in Organic Chemistry, ed J. F.W. McOmie, Plenum Press, 1973; T.W. Greene & P.G.M. Wuts, Protective Groups in Organic Chemistry, John Wiley & sons, 1991]. The protective groups can be eliminated in a suitable latter stage by methods known to those skilled in the art.

If the sulphonamide derivatives of general.formula (I) are obtained in form of a mixture of stereoisomers, particularly enantiomers or diastereomers, said mixtures may be separated by standard procedures known to those skilled in the art, e.g. chromatographic methods or crystallization with chiral reagents.

The medicament obtained according to the present invention is particularly suitable for the administration to mammals, including humans.The medicament may preferably be administered to humans of all age groups, i.e. children, adolescents as well as adults.

A further aspect of the present invention is the use of at least one sulphonamide derivative of above given general formula (I), optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate, for the manufacture of a medicament for the regulation of appetite, for the reduction, increase or maintenance of body weight, for the prophylaxis and/or treatment of obesity, bulimia, anorexia, cachexia or type II diabetes, preferably type II diabetes caused by obesity.

Particularly preferred is the use of at least one sulphonamide derivative of above given general formula (I), optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate, for the manufacture of a medicament for the prophylaxis and/or treatment of obesity.

The preparation of corresponding pharmaceutical compositions as well as of the formulated medicaments may be carried out by conventional methods known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986).

The pharmaceutical compositions as well as the formulated medicaments prepared according to the present invention may in addition to at least one sulphonamide derivative of general formula (I), optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate, comprise further conventional auxiliary substances known to those skilled in the art, such as carriers, fillers, solvents, diluents, colouring agents, coating agents, matrix agents and/or binders. As is also known to those skilled in the art, the choice of the auxiliary substances and the amounts thereof to be used are dependent on the intended route of administration, e.g. oral, rectal, intravenous, intraperitoneal, intramuscular, intranasal, buccal or topical route.

Medicaments suitable for oral administration are for example, tablets, sugar-coated pills, capsules or multiparticulates, such as granules or pellets, optionally compressed into tablets, filled into capsules or suspended in a suitable liquid, solutions or suspensions.

Medicaments suitable for parenteral, topical or inhalatory administration may preferably be selected from the group consisting of solutions, suspensions, readily reconstitutable dry preparations and also sprays.

Suitable medicaments, e.g. medicaments for oral or percutaneous use may release the sulphonamide compounds of general formula (I) in a delayed manner, whereby the preparation of these delayed release medicaments is generally known to those skilled in the art.

Suitable delayed-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728.

The medicament of the present invention may also have at least one enteric coating which dissolves as a function of pH. Because of this coating, the medicament can pass through the stomach undissolved and the compounds of general formula I are only released in the intestinal tract. The enteric coating preferably dissolves at a pH of between 5 and 7.5. Suitable materials and methods for the preparation of enteric coatings are also known to those skilled in the art

Typically the pharmaceutical compositions and medicaments comprise 1 to 60 % by weight of one or more sulphonamide derivatives of general formula (I) and 40 to 99 % by weight of one or more excipients.

The amount of active ingredient to be administered to the patient varies in dependence on the weight of the patient, the route of administration, the indication and the degree of severity of the disorder. Usually 1 to 5000, preferably 1 to 2500, more preferably 1 to 500 mg of at least one sulphonamide derivative of general formula (I) are administered to the patient in need of treatment per day. The total daily dose may be administered to the patient in one or more portions.

### Pharmacological Methods:

### BINDING TO SEROTONIN RECEPTOR 5HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytriptamine-7 Receptors. The Journal of Pharmacology and Experimental Therapeutics, 1994, 268, 1403] with the following slight changes.
The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220

### FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the inventively used sulphonamide derivatives of general formula (I) on food intake in fasted rats is then determined as follows:

The rats were fasted for 23 hours in their single homecages. After this period, the rats are orally or intraperitoneally dosed with a composition comprising a sulphonamide derivative of general formula (I) or a corresponding composition (vehicle) without said sulphonamide derivative. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and of Turnbull et al., Diabetes, Vol. 51, August 2002.

The present invention is illustrated below with the aid of examples. These illustrations are given solely by way of example and do not limit the general spirit of the present invention.

### Examples:

### METHOD A

### Example 7:

### Preparation of N-[3-(2-dimethylaminoethyl)-1H-indol-5-yl]-5-chloro-3-methyl-benzo[b]thiophene-2-sulphonamide.

To a solution of 3.05 g (15 mMol) of 5-amino-3-(2-dimethylaminoethyl)-1*H*-indol in 100 ml of pyridine is added dropwise at ambient temperature a solution of 4.21 g (15 mMol) of 5-chloro-3-methyl-benzo[b]thiophene-2-sulphonyl chloride in 20 ml of pyridine. The reaction mixture is stirred at ambient temperature for 20 hours. It is then evaporated to dryness, slightly alkalinised with diluted ammonia and dissolved in ethyl acetate. The organic phase is washed with water and a saturated solution of sodium bicarbonate, it is separated and dried with anhydrous sodium sulphate. The organic solution is evaporated to dryness and the resulting solid is repeatedly washed with ethyl ether, to yield 5.5 g (82%) of N-[3-(2-dimethylaminoethyl)-1*H*-indol-5-yl]-5-chloro-3-methyl-benzo[b]thiophene-2-sulphonamide as a solid with m.p. = 226-227°C.

### METHOD B

### Example 26:

### Preparation of N-[3-(2-diethylaminoethyl)-1H-indol-5-yl]-N-ethyl-naphthalene-2-sulphonamide.

To a mixture of 285 mg (0.7 mMol) of N-[3-(2-diethylaminoethyl)-1H-indol-5yl]naphthalene-2-sulphonamide (example 17) and 80 mg (0.7 mMol) of potassium t-butoxide in 3 ml of DMSO are stirred for 30 minutes at ambient temperature.

Then are added 105 mg (0.7 mMol) of ethyl iodide and left with stirring for 3 hours. Water is added and is extracted with ethyl acetate. The organic solution is evaporated to dryness and the resulting crude is purified by chromatography on silica gel, using as an eluent mixtures of methylene chloride / methanol /ammonia, yielding N-[3-(2-diethylaminoethyl)-1*H*-indol-5-yl]-N-ethylnaphthalene-2-sulphonamide as a solid with m.p. = 49-50°C.

### METHOD E

### Example 3:

### Preparation of N-[3-(2-diethylaminoethyl)-1H-indol-5-yl]naphthalene-1-sulphonamide hydrochloride.

1.05 g (2.5 mMol) of N-[3-(2-diethylaminoethyl)-1*H*-indol-5-yl]naphthalene-1-sulphonamide (example 2) are dissolved in 10 ml of ethanol and 0.6 ml are added of a 4.2 N solution of hydrochloric acid in ethanol. It is allowed to crystallise at ambient temperature. N-[3-(2-diethylaminoethylyl)-1*H*-indol-5-yl]naphthalene-1-sulphonamide hydrochloride is obtained as a solid with m.p.= 255-257°C.

The melting point and spectroscopic data for identifying some of the compounds used according to the present invention are shown in the following table:

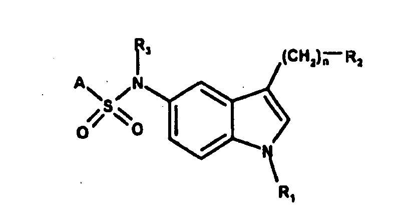

| Ex | R₁ | R₂ | n | R₃ | A | Salt | m.p. °C | IR cm⁻¹ | ¹H-RMN (300 MHz),δ (solvent) |
|---|---|---|---|---|---|---|---|---|---|
| 1 | H | (CH₃CH₂)₂N- | 2 | H | 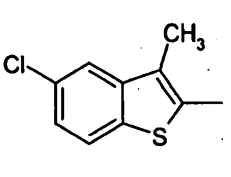 | - | 170-173 | 3387, 2970, 2931,1466, 1236, 1158, 1107, 1080, 993, 862, 805, 657, 565. | 0.88(t, 6H, J=7.1 Hz); 2.28(s, 3H); 2.30-2.46(m, 6H); 2.58(m, 2H); 6.85(dd, 1H, J=8.6, 2.0 Hz); 7.10(m, 2H); 7.20(d, 1H, J=8.6 Hz); 7.50(dd, 1H, J=8.7, 2.0 Hz); 7.90(d, 1H, J=2.0 Hz); 7.98(d, 1H, J=8.7 Hz); 10.10 (bb, 1H); 10.80(s, 1H). (DMSO-d6) |
| 2 | H | (CH₃CH₂)₂N- | 2 | H | 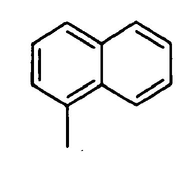 | - | 170 | 3451, 3337, 2972, 1466, 1319, 1237, 1157, 1132, 1091, 991, 770, 675, 583, 481. 675, 583, 481. | 0.90(t, 6H, J=7.1 Hz); 2.33-2,55(m, 8H); 6.69(dd, 1H, J=8.7, 1.8 Hz); 6, 95(s, 1H); 7,02(d, 1H, J=1,8 Hz); 7.05(d, 1H, J=8.7 Hz); 7.47(t, 1H, J=7.7 Hz); 7.63(m, 1H); 7.70(m, 1H); 8.01 (m, 2H); 8.12(d, 1H, J=7.5 Hz); 8.77(d, 1H, J=8.1 Hz); 10.10(bb, 1H); 10,66(s, 1H) (DMSO-d6) |
| 3 | H | (CH₃CH₂)₂N- | 2 | H | 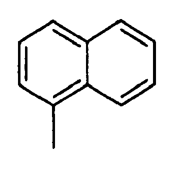 | HCl | 255-257 | 3378, 3065, 2558, 2489, 1460, 1317, 1162, 1143, 1131, 811, 687, 602, 588. | 1.22(t, 6H, J=7.2 Hz); 2.91-3.18(m, 8H); 6.65(d, 1H, J=8.6 Hz); 7.08(d, 1H, J=8.6 Hz); 7.17(s, 1H); 7.20(d, 1H, J=1.8 Hz); 7.54(t, 1H, J=7.8 Hz); 7.63(m, 1H); 7.70(m, 1H); 8.03(d, 1H, J=7.8 Hz); 8.08(d, 1H, J=7.1 Hz); 8.14(d, 1H, J=8.2 Hz); 8.79(d, 1H, J=8.4 Hz); 10.26(s, 1 H); 10.90(bb, 1H); 11.01(s, 1H). (DMSO-d6) |
| 4 | H | (CH₃CH₂)₂N- | 2 | H | 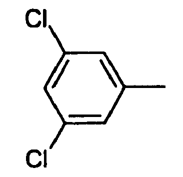 | - | 168-170 | 3309, 3047, 2974, 1566, 1467, 1235, 1167 1143, 1116, 1001, 910, 799, 672, 587. | 0.95(t, 6H, J=7.1 Hz); 2.44-2.58(m, 6H); 2.66(m, 2H); 6.79(dd, 1H, J=8.6, 1.7 Hz); 7.08(d, 1H, J=0.9 Hz); 7.13(d, 1H, J=1.7 Hz); 7.23(d, 1H, J=8.6 Hz); 7.58 (m, 2H); 7.87(m, 1 H); 9,95(bb, 1H); 10.82(s, 1H). (DMSO-d6) |
| 5 | H | (CH₃CH₂)₂N- | 2 | H | 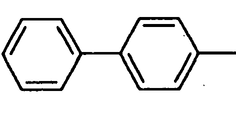 | - | 161-163 | 3387, 2971, 1323, 1157, 1095, 765, 670, 590 | 0.89(t, 6H, J=7.1 Hz); 2.32-2.55(m, 6H); 2.62(m, 2H); 6.85(d, 1H, J=8.6 Hz); 7.08(d, 1H, J=2.0 Hz); 7.13(s, 1H); 7.18(d, 1H, J=8.6 Hz); 7.33-750 (m, 3H); 7.64(d, 2H, J=7.5 Hz); 7.72(sys AB, 2H, J=8.6 Hz); 7,78(sys AB, 2H, J=8.6 Hz); 9.80(bb, 1H); 10.75(s, 1H). (DMSO-d6) |
| 6 | H | (CH₃CH₂)₂N- | 2 | H | 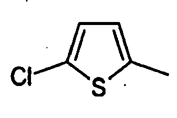 | - | 180-181 | 3375, 2978, 1467, 1417, 1236, 1212, 1115, 994, 624. | 0.96(t, 6H, J=7.1 Hz); 2.52(m, 4H); 2.57(m, 2H); 2.66(m, 2H); 6.83(dd, 1H, J=8.6, 1.9 Hz); 7.11(d, 1H, J=4.0 Hz); 7.14(d, 1H, J=1.9 Hz); 7.17(d, 1H, J=1.9 Hz); 7.20-7.24(m, 2H); 10.01(bb, 1 H); 10.81(s, 1H). (DMSO-d6) |
| 7 | H | (CH₃)₂N- | 2 | H | 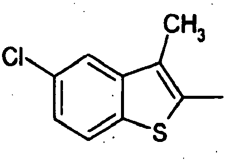 | - | 226-227 | 3422, 3238, 1332, 1155, 1114; 1079, 986, 861, 803, 655, 564. | 2.04(s, 6H); 2.23(m, 2H); 2.28(s, 3H), 2.59(m, 2H); 6.83(dd, 1H, J=8.4, 1.5 Hz); 7,09 (s, 2H); 7.19(d, 1H, J=8.4 Hz); 7.49(dd, 1H, J=8.7, 1.6 Hz); 7.91(d, 1H, J=1.6 Hz); 7.99(d, 1H, J=8.7 Hz); 10,13(bb, 1H), 10,79 (s, 1H) (DMSO-d6) |
| 8 | H | (CH₃)₂N- | 2 | H | 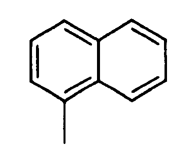 | - | 203-205 | 3357, 1475, 1282, 1157, 1127, 990, 957, 809, 773, 613, 587, 557, 498. | 2.09(s, 6H); 2.21(m, 2H); 2.54(m, 2H); 6.69(dd, 1H, J=8.6, 1.7 Hz); 6,94 (s, 1H); 7.03 (s, 1 H); 7.06(d, 1H, J=8.1 Hz); 7.49(t, 1H, J=7.8 Hz); 7.64(m, 1H); 7.71(m, 1H); 8.02 (m, 2H); 8.13(d, 1H, J=8.1 Hz); 8.79(d, 1H, J=8.4 Hz); 10.10(bb, 1H); 10.68(s, 1H) (DMSO-d6) |
| 9 | H | (CH₃)₂N- | 2 | H | 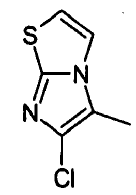 | - | 215 (desc) | 3247, 3094, 1467, 1272, 1261, 1230, 625 | 2.17(s, 6 H); 2.36(m, 2 H); 2.65(m, 2 H); 6.77(dd, J=8.6, 1.7 Hz, 1 H); 7.07(s, 1 H); 7.09(s, 1H); 7.18(d, J=8.6 Hz, 1 H); 7.51(d, J=4.5 Hz, 1 H); 7.81(d, J=4.5 Hz, 1 H); 10.80 (s, 1H). (DMSO-d6). |
| 17 | H | (CH₃CH₂)₂N- | 2 | H | 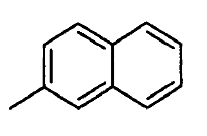 | - | 172-173 | 3199, 2970, 2930, 2870, 1327, 1153, 1130, 1110, 1075, 956, 676, 658, 551, 476. | 0.87(t, J=7.1 Hz, 6 H); 2.39(m, 6 H); 2.55 (m, 2 H); ); 6.82(d, J=8.6 Hz, 1 H); 7.05 (s, 1 H); 7.09(s, 1 H); 7.13(d, J=8.6 Hz, 1 H); 7.60(m, 2 H); 7.73 (d, J=8.6 Hz, 1 H); 7.95(d, J=7.9 Hz, 1 H) 8.01 (m, 2 H); 8.26 (s, 1 H); 9.86(bb, 1 H); 10.71(s, 1 H). (DMSO-d6). |
| 19 | H | 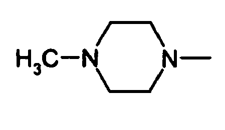 | 1 | H | 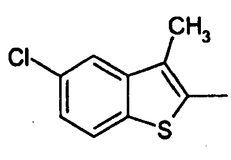 | - | 230 (desc) | 2796, 1452, 1316, 1149, 1114, 1080, 1001, 810, 646, 559. | 1.80-2.26(m, 8 H); 2.04(s, 3 H); 2.30(s, 3 H); 3.41(s, 2 H); 6.89(dd, J=8.6, 1.56 Hz, 1 H); 7.16(s, 1 H); 7.22(d, J=8.6 Hz, 1 H); 7.29(s, 1 H); 7.49(dd, J=8.7, 1.7 Hz, 1 H); 7.90(d, J=1.7 Hz, 1 H); 7.98(d, J=8.7 Hz, 1 H); 10.13(bb, 1 H); 10.93(s, 1 H). (DMSO-d6). |
| 20 | H | (CH₃)₂N- | 2 | H | 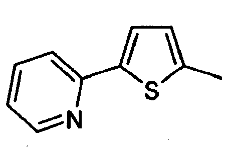 | - | 209-211 | 3377, 2951,2798, 1469, 1429, 1321, 1158, 777, 594. | 2.05(s, 6 H); 2.32(m, 2 H); 2.65(m, 2 H); 6.86(dd, J=8.6, 1.8 Hz, 1 H); 7.10(d, J=1.8 Hz, 1 H); 7.18(d, J=1.8 Hz, 1 H); 7.21 (d, J=8.6 Hz, 1 H); 7.32(dd, J=7.5, 4.6 Hz, 1 H); 7.36(d, J=3.9 Hz, 1 H); 7.71(d, J=3.9 Hz, 1 H); 7.83(m, 1 H); 7.93(m, 1 H); 8.49(d, J=4.6 Hz, 1 H); 9.97(bb, 1 H); 10.79(s, 1 H). (DMSO-d6). |
| 21 | H | (CH₃)₂N- | 2 | H | 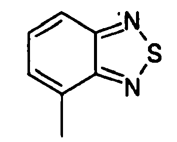 | - | 192 | 3321, 2949, 1474, 1327, 1152, 1138, 1104, 981, 614. | 2.10(s, 6 H); 2.21(m, 2 H); 2.56(m, 2 H); 6.72(d, J=8.6 Hz, 1 H); 6.96(s, 1 H); 7.03 (s, 1 H); 7.07(d, J=8.6 Hz, 1 H); 7.70(m, 1 H); 8.07(d, J=7.0 Hz, 1 H); 8.29(d, J=8.8 Hz, 1 H); 10.14(bb, 1 H); 10.69(s, 1 H). (DMSO-d6). |
| 22 | H | (CH₃)₂N- | 2 | H | 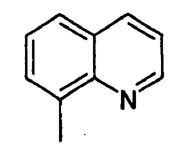 | - | 250 (desc) | 3252, 2857, 1459, 1426, 1333, 1161, 1144, 789, 680, 589. | 2.07(s, 6 H); 2.16(m, 2 H); 2.51 (m, 2 H); 6.73(dd, J=8.6, 1.8 Hz, 1 H); 6.94(s, 1 H) 6.99(s, 1 H); 7.02(d, J=8.6 Hz, 1 H); 7.59(t, J=7.8 Hz, 1 H); 7.73(dd, J=8.4, 4.1 Hz, 1 H); 8.18(m, 2 H); 8.50(dd, J=8.4, 1.5 Hz, 1 H); 9.20(dd, J=4.1, 1.5 Hz, 1 H); 9.45(bb, 1 H); 10.64(s, 1 H). (DMSO-d6). |
| 23 | H | (CH₃)₂N- | 2 | H | 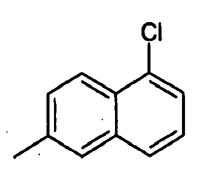 | - | 230-240 (desc) | 3404, 2944, 2918, 2855, 1465 1332, 1157, 1140, 1080, 650, 639, 526. | 2.01(s, 6 H); 2.18(m, 2 H); 2.57(m, 2 H); 6.81 (dd, J=8.6, 1.7 Hz, 1 H); 7.02 (s, 1 H); 7.05(d, J=1.7 Hz, 1 H); 7.15(d, J=8.6 Hz, 1 H); 7.57(m, 1 H); 7.82(d, J=7.5 Hz, 1 H); 7.91(d, J=8.9 Hz, 1 H); 8.06(d, J=8.2 Hz, 1 H); 8.29(d, J=8.9 Hz, 1 H); 8.35(s, 1 H); 9.94(bb, 1 H); 10.74(s, 1 H). (DMSO-d6). |
| 24 | H | (CH₃)₂N- | 2 | H | 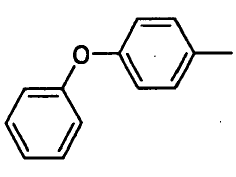 | - | 152-154 | 3232, 2862, 2827, 2785, 1583, 1488, 1333, 1248, 1155, 1091, 755, 693, 571, 541. | 2.16(s, 6 H); 2.37(m, 2 H); 2.66 (m, 2 H); 6.80 (d, J=8.6 Hz, 1 H); 6.96-7.12 (m, 6 H); 7.14-7.25 (m, 2 H); 7.12 (m, 6 H); 7.14-7.25 (m, 2 H); 7.41 (m, 2 H); 7.64 (dd, J=8.5, 1.9 Hz, 2 H); 9.69(bb, 1 H); 10.75 (s, 1 H). (DMSO-d6). |
| 25 | H | (CH₃)₂N- | 2 | H | 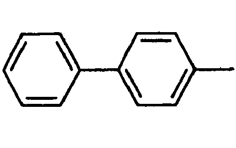 | - | 184-186 | 3451, 3388, 2950, 2775, 1466, 1322, 1159, 1095, 763, 670, 591. | 2.08(s, 6 H); 2.32(m, 2 H); 2.64(m, 2 H); 6.83(dd, J=8.6, 1.9 Hz, 1 H); 7.08(d, J=2.0 Hz, 1 H); 7.11(d, J=1.9 Hz, 1H); 7.17(d, J=8.6 Hz, 1 H); 7.34-7.50(m, 3H); 7.66(d, J=7.5 Hz, 2 H); 7.72(AB sys, J=8.6 Hz, 2 H); 7.79(AB sys, J=8.6 Hz, 2 H); 9.79(s, 1 H); 10.75(s, 1 H). (DMSO-d6). |
| 26 | H | (CH₃CH₂)₂N- | 2 | Et | 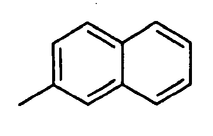 | - | 49-50 | 3386, 2970, 2931, 1474, 1337,1167, 1151, 1130, 1073, 661, 550 | 0.82(t, J=7.0 Hz, 6 H); 0.98(t, J=7.0 Hz, 3 H); 2.37(q, J=7.0 Hz, 4 H); 2.49(m, 2 H); 2.54(m, 2H); 3.66(q, J=7.1 Hz, 2 H); 49-50 6.73 (dd, J=8.61, 1.6 Hz, 1 H); 6.98(s, 1 H); 7.17 (d, J=1.6 Hz, 1 H); 7.26(d, J=8.61 Hz, 1 H); 7.56-7.72 (m, 3 H); 7.99-8.11(m, 3H); 8.26 (s, 1 H); 10.97(s, 1 H). (DMSO-d6). |
| 27 | H | 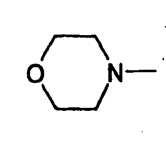 | 2 | H | 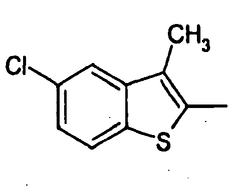 | - | 200-201 | 3366, 2951, 2816, 1460, 1421, 1319, 1283, 1114, 1078, 865, 651, 561 | 2.25(m, 6H); 2.27(s, 3H); 2.62(t, J=7.9 Hz, 2H); 3.52(m, 4H); 6.84(d, J=8.2 Hz, 1H); 7.06(s, 1H); 7.10(s, 1H); 7.20(d, J=8.6 Hz, 1H); 7.50(d, J=8.6 Hz, 1H), 7.92(s, 1H); 8.00 (d, J=8.6 Hz, 1H); 10.13(s, 1H); 10.80(s, 1H). (DMSO-d6) |
| 28 | H | 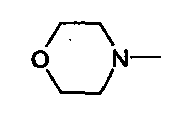 | 2 | H | 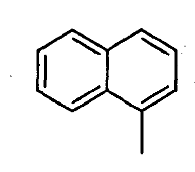 | - | 218-220 | 3389, 3152, 2916, 2819, 1466, 1313, 1157, 1129, 1108, 771, 587 | 2.30(m, 6H); 2.56(m, 2H); 3.56(m, 4H); 6.69(d, J=8.4 Hz, 1H); 6.93(s, 1H); 7.06(m, 2H); 7.48(t, J=7.3 Hz, 1H); 7.67(m, 2H); 8.02(m, 2H); 8.13 (d, J=8.1 Hz, 1 H); 8.78 (d, J=8.1 Hz, 1H); 10.10(s, 1H); 10.68(s, 1H). (DMSO-d6) |
| 29 | H | (CH₃CH₂)₂N- | 2 | CH₃ | 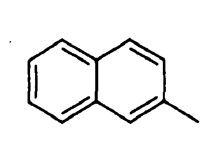 | - | 134-136 | 2968, 2930, 1488, 1329, 1159, 1131, 550 | 0.98(t, J=7.1 Hz, 6H); 2.55(m, 6H); 2.70(m, 2H); 3.67(s, 3H); 6.84 (s. 1H); 6.93(dd, J=8.6, 2 Hz, 1H); 7.10(d, J=8.7 Hz, 1H); 7.18(d, J=1.7 Hz, 1H); 7.26(s, 1H); 7.57 (m, 2H); 7.67(dd, J=8.7, 1.8 Hz, 1H); 7.84(m, 3H); 8.27(d, J= 1.7 Hz, 1H). (DMSO-d6) |
| 30 | H | (cH₃)₂N- | 1 | H | 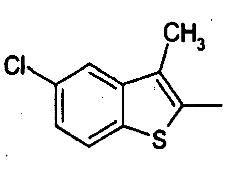 | - | 148-152 | 3398, 2930, 1467, 1158, 1113, 1079, 861, 803,651, 561 | 1.89(m, 6H); 2.29(s, 3H); 2.48(s, 2H); 6.83(m, 1H); 7.18(m, 3H); 7.50(m, 1H); 7.91(m, 1H); 8.00 (m, 1H); 10.13(b, 1H); 10.92(s, 1H). (DMSO-d6) |
| 31 | H | (CH₃cH₂CH₂)₂N- | 2 | H | 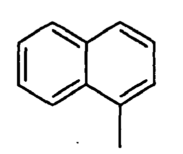 | - | 76-80 | 3399, 2959, 2931, 1466, 1159, 1132, 802, 770, 588 | 0.82(t, J=6.7 Hz, 6H); 1.34(q, J=6,71 Hz, 4H); 2.31(m, 4H); 2.40(m, 2H); 2.52(m, 2H); 6.69(d, J=8.6 Hz, 1H); 7.04(m, 3H); 7.47(m, 1H); 7.66(m, 2H); 8.02(m, 2H); 8.11(d, J=8.1 Hz, 1H); 8.78(d, J=8.4 Hz, 1H); 10.12(s, 1H); 10.67(s, 1 H). (DMSO-d6) |
| 32 | H | (CH₃CH₂CH₂)₂N- | 2 | H | 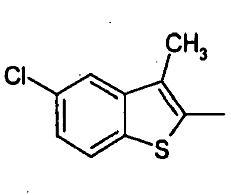 | - | 90-95 | 3406, 2959, 2932, 2872, 1466, 1157, 1079, 861, 652, 561 | 0.80(t, J=7.3 Hz, 6H); 1.31(q, J=7.3 Hz, 4H); 2.26(m, 7H); 2.38(m, 2H); 2.56(m, 2H); 6.83(dd, J=8.4, 1.8 Hz, 1H); 7.08(s, 2H); 7.20(d, J=8.6 Hz, 1 H); 7.50(dd, J=8.6, 2.0 Hz, 1H); 7.90(d, J=2.0 Hz, 1H); 7.99(d, J=8.6 Hz, 1H); 10.12(b, 1H); 10.79(s, 1 H). (DMSO-d6) |
| 33 | H | (CH₃CH₂CH₂CH₂)₂N- | 2 | H | 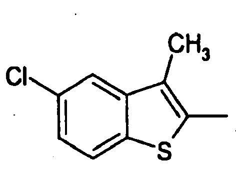 | - | 79-80 | 3398, 2956, 2930, 2870, 1466, 1158, 1080, 862, 801, 653, 562 | 0.84(t, J=6.8 Hz, 6H); 1.24(m, 8H); 2.26(s, 3H); 2.28(m, 4H); 2.39(m, 2H); 2.57(m, 2H); 6.82(dd, J=8.6, 1.9 Hz, 1H); 7.09(d, J=1.8 Hz, 2H); 7.18(d, J=8.6 Hz, 1H); 7.50(dd, J=8.6, 1.9 Hz, 1H); 7.89(d, J=1.8 Hz, 1H); 7.98(d, J=8.6 Hz, 1H); 10.14(b, 1H); 10.78(s, 1 H). (DMSO-d6) |
| 34 | H | (CH₃CH₂CH₂CH₂)₂N- | 2 | H | 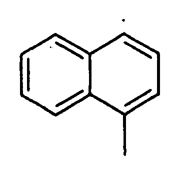 | - | 111-113 | 3291, 2955, 2926, 2870, 1327, 1158, 1136, 772, 676, 611, 585 | 0.86(t, J=7.0.Hz, 6H); 1.29(m, 8H); 2.35(m, 4H); 2.41(m, 2H); 2.53(m, 2H); 6.67(dd, J=8.5, 1.9 Hz, 1 H); 7.09(m, 3H); 7.48(t, J=7.9 Hz, 1H); 7.68(m, 2H); 8.01(s, , 1H); 8.04(s, 1H); 8.12(d, J=8.2 Hz, 1H); 8.78(d, J=8.2 Hz, 1H); 10.13(s, 1H); 10.67(s, 1H). (DMSO-d6) |
| 35 | H | (CH₃CH₂)₂N- | 2 | H | 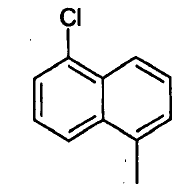 | - | 154-156 | 3402, 2978, 1471, 1285, 1162, 1135, 1018, 780, 629, 606 | 0.88(t, J=6.7 Hz, 6H); 2.41(m, 6H); 2.49(m, 2H); 6.71(d, J=8.1 Hz, 1H); 6.88(s, 1H); 7.07(m, 2H); 7.66(m, 2H); 7.84(d, J=7.0 Hz, 1H); 8.09(d, J=7.0 Hz, 1H); 8.41(d, J=8.2 Hz, 1H); 8.79(d, J=8.6 Hz, 1H); 10.17(b, 1H); 10.71(s, 1 H). (DMSO-d6) |
| 36 | H | (CH₃CH₂)₂N- | 2 | H | 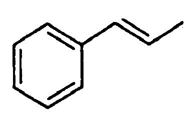 | - | 125-130 | 3404, 2972, 1473, 1319, 1142,967, 745, 541 | 0.94(t, J=7.1 Hz, 6H); 2.50(q, J=7.1 Hz, 4H); 2.59(m, 2H); 2.68(m, 2H); 6.94(dd, J=8.6, 1.8 Hz, 1H); 7.26(m, 8H); 7.59(m, 2H); 9.54(b, 1H); 10.77(s, 1H). (DMSO-d6) |
| 37 | H | 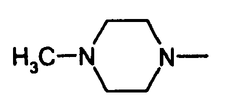 | 1 | H | 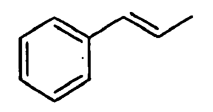 | - | 203 (desc) | 2809, 1340, 1150, 746, 542 | 2.06(s, 3H); 2.22(m, 6H); 3.36(m 2H); 3.49 (s, 2H); 6.95(dd, J=8.6, 1.8 Hz, 1H); 7.18(s, 2H); 7.24(m, 2H); 7:37(m, 3H); 7.45(d, J=1.8 Hz, 1H); 7.61(m, 2H); 9.53(s 1H); 10.90(s, 1H). (DMSO-d6) |
| 39 | H | (CH₃CH₂)₂N- | 2 | H | 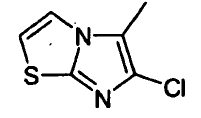 | - | 197-198 | 3338, 1466, 1270, 1237, 117, 986, 626 | 0.96(t, J=7.1 Hz, 6H); 2.53(m, 6H); 2.63(m, 2H); 6.78(dd, J=8.5, 1.6 Hz, 1H); 7.10(s, 2H); 7.18(d, J=8.6 Hz, 1H); 7.51 (d, J=4.6 Hz, 1H); 7.80(d, J=4.6 Hz, 1H); 10.78(s, 1 H). (DMSO-d6) |
| 40 | H | 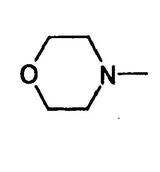 | 2 | H | 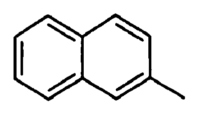 | - | 85-90 | 3399, 3257, 2920, 2855, 2814, 1460, 1330, 1157, 1131, 1113, 1074, 659, 551,477 | 2.27(m, 6H); 2.61(t, J=7.9 Hz, 2H); 3.52(t, J=4.6 Hz, 4H); 6.82(dd, J=8.6, 2.0 Hz, 1H); 7.06(s, 1H); 7.07(s, 1H); 7.15(d, J=8.6 Hz, 1H); 7.61(m, 2H); 7.74(dd, J=8.8, 1.8 Hz, 1H); 7.96(d, J=8.1 Hz, 1H); 8.03(m, 2H); 8.27 (s, 1H); 9.87(s, 1H); 10.74(s, 1H). (DMSO-d6) |
| 41 | H | 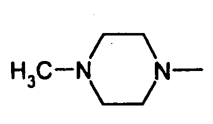 | 1 | H | 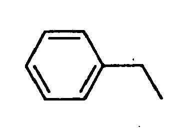 | - | 99-102 | 3398, 2934, 2806, 1458, 1331, 1284, 1153, 1127, 700, 542 | 2.11(s, 3H); 2.32(m, 6H); 3.35(m, 2H); 3.56(s, 2H); 4.29(s, 2H); 6.98(d, J=8.2 Hz, 1H); 7.29(m, 7H); 7.53(s, 1H); 9.40(s, 1H); 10.94(s, 1H). (DMSO-d6) |
| 42 | H | (CH₃CH₂)₂N- | 3 | H | 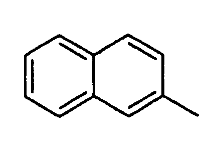 | - | 128-130 | 3259, 2973, 2827, 1468, 1332, 1159, 1131, 1075, 670, 555 | 0.86(t, J=7.0 Hz, 6H); 1.51(t, J=6.9 Hz, 2H); 2.27(t, J=6.9 Hz, 2H); 2.35(q, J=7.0 Hz, 4H); 2.46(m, 2H); 6.77(d, J=8.6Hz, 1H); 7.00(s, 1H); 7.10(m, 2H); 7.60(m, 2H); 7.72(d, J=8.8 Hz, 1H); 7.95(d, J=7.9 Hz, 1H); 8.02(m, 2H); 8.26(s, 1H); 9.86 (b, 1H); 10.67(s, 1H). (DMSO-d6) |
| 43 | H | (CH₃CH₂)₂N- | 3 | H | 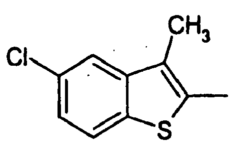 | - | 156-158 | 3247, 2969, 2938, 1467, 1340, 1159, 1113, 1080, 862, 666, 558 | 0.88(t, J=7.0 Hz, 6H); 1.52(m, 2H); 2.29(m, 5H); 2,37(q, J=7.0 Hz, 4H); 2.47(m, 2H); 6.81 (dd, J=8.6, 1.5 Hz, 1 H); 7.06(d, J=1.6 Hz, 1H); 7.12(d, J=1.5 Hz, 1H); 7.18(d, J=8.6 Hz, 1H); 7.51 (dd, J=8.6, 2.0 Hz, 1H); ); 7.91(d, J=2.0 Hz, 1H); 7.99(d, J=8.6 Hz, 1H);10.06(b, 1H); 10.76(s, 1 H). (DMSO-d6) |
| 44 | H | 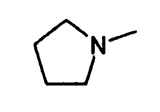 | 2 | H | 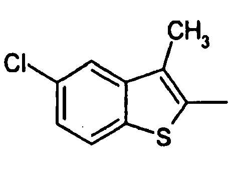 | - | 201-203 | 3386, 2929, 1466, 1157, 1106, 861, 650, 564 | 1.62(m, 4H); 2.29(s, 3H); 2.30(m, 4H); 2.36(m, 2H); 2.63(m, 2H); 6.86(d, J=8.6 Hz, 1H); 7.05(s, 1H); 7.09(s, 1H); 7.21(dd, J=8.6, 2.2 Hz, 1H); 7.50(dd, J=8.7, 2.0 Hz, 1H); 7.92(s, 1H); 7.99(dd, J=8.7, 2.2 Hz, 1H); 10,10(b, 1H); 10.81(s, 1H). (DMSO-d6) |
| 45 | H | 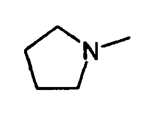 | 2 | H | 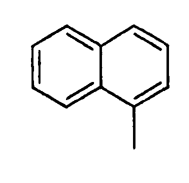 | - | 212-214 | 3354, 2964, 2812, 1466, 1201, 1157, 1124, 808, 773, 593 | 1.66(m, 4H); 2.36(m, 6H); 2.58(m, 2H); 6.71(d, J=8.6 Hz, 1H); 6.93(s, 1H); 7.02(s, 1H); 7.07(d, J=8.6 Hz, 1H); 7.48 (m, 1H); 7.68(m, 2H); 8.02(dd, J=7.2, 1.2 Hz, 2H); 8.12(d, J=8.2 Hz, 1H); 8.79(d, J=8.6 Hz, 1H); 10.10(b, 1H); 10.68(s, 1H). (DMSO-d6) |
| 46 | H | 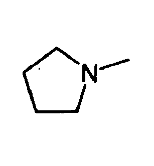 | 2 | H | 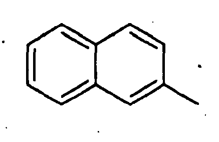 | - | 180-182 | 3375, 2968, 2821, 1467, 1323, 1313, 1146, 1139, 1131, 1079, 972, 654, 549 | 1.60(m, 4H); 2.26(m, 4H); 2.35(m, 2H); 2.61 (m, 2H); 6.82(dd, J=8.6, 2.0 Hz, 1 H); 7.05(m, 2H); 7.14(d, J=8.6 Hz, 1H); 7.61(m, 2H); 7.74(dd, J=8.6, 1.8 Hz, 1H); 7.95(d, J=7.9 Hz, 1H); 8.02(m, 2H); 8.27(s, 1H); 9.86(b, 1H); 10.72(s, 1H). (DMSO-d6) |
| 47 | H | (CH₃CH₂CH₂)₂N- | 2 | H | 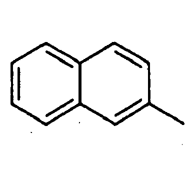 | - | 58-64 (desc) | 3398, 3255, 2958, 2931, 2872, 1466, 1330, 1156, 1130, 1074, 659, 551 | 0.79(t, J=7.3 Hz, 6H); 1.31(q, J=7.3 Hz, 4H); 2.28(t, J=7.3 Hz, 4H); 2.42(m, 2H); 2.57(m, 2H); 6.80(dd, J=8.6, 1.7 Hz, 1H); 7.04(d, J=1.7 Hz, 1H); 7.12(m 2H); 7.60(m, 2H); 7.72(dd, J=8.6, 1.7 Hz, 1H); 7.98(m, 3H); 8.25(s, 1H); 9.87(b, 1H); 10.70(s, 1H). (DMSO-d6) |
| 48 | H | (CH₃)₂N- | 2 | H | 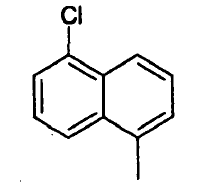 | - | 201-203 | 3369, 1473, 1161, 1125, 1017, 619 | 2.06(s, 6H); 2.15(t, J=8.2 Hz, 2H); 2.52(t, J=8.2 Hz, 2H); 6.69(d, J=8.7 Hz, 1H); 6.85(s, 1H); 7.02(s, 1H); 7.08(d, J=8.7 Hz, 1H); 7.67(m, 2H); 7.84(d, J=7.3 Hz, 1H); 789, 810(d, J=7.3 Hz, 1H); 8.41(d, J=8.4 Hz, 1H); 8.79(d, J=8.7 Hz, 1H); 10.15(b, 1H); 10.70(s, 1H). (DMSO-d6) |
| 49 | H | (CH₃)₂N- | 2 | H | 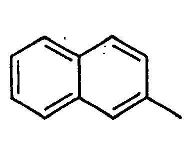 | - | 180-190 | 3399, 3255, 2943, 1466, 1330, 1156, 1131, 1075, 659, 550 | 2.03(s, 6H); 2.22(t, J=8.2 Hz, 2H); 2.58(t, J=8.2 Hz, 2H); 6.80(d, J=8.4 Hz, 1H); 7.04(s, 1H); 7.07(s, 1 H); 7.13(d, J=8.6 Hz, 1H); 7.60(m, 2H); 7.74(d, J=8.6 Hz, 1H); 7.95(d, J=7.7 Hz, 1H); 8.02(m, 2H); 8.26(s, 1H); 9.86(b, 1H); 10.71(s, 1H). (DMSO-d6) |
| 50 | H | 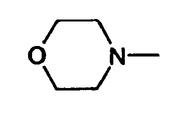 | 2 | H | 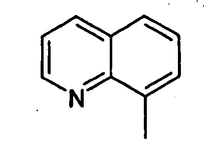 | - | 234-235 | 3400, 3279, 2913, 2852, 1464, 1420, 1315, 1163, 1118, 951, 592 | 2.29(m, 6H); 2.54(m, 2H); 3.57(m, 4H); 6.72(d, J=8.1 Hz, 1H); 7.01(m, 3H); 7.60(t, J=7.7 Hz, 1H); 7.74(d, J=8.4 Hz, 1H); 8.19(m, 2H); 8.52(d, J=8.4 Hz, 1H); 9.21(s, 1H); 9.44(s, 1H); 10.65(s, 1H). (DMSO-d6) |
| 51 | H | 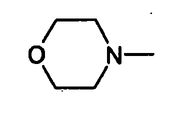 | 2 | H | 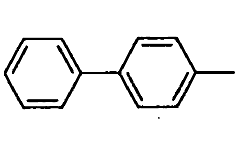 | - | 225-228 | 3340, 2857, 1479, 1324, 1153, 1116, 1094, 768, 670, 588 | 2.29(m, 6H); 2.66(m, 2H); 3.47(m, 4H); 6.84(d, J=8.6 Hz, 1H); 7.07(s, 1H); 7.09(s, 1H); 7.18(d, J=8.4 Hz, 1H); 7.45(m, 3H); 7.70(m, 4H); 7.79(m, 2H); 9.79(s, 1H); 10.77(s, 1H). (DMSO-d6) |

### Example 54:

Tablet comprising an inventively used sulphonamide compound of general formula I Formula per tablet:

| | |
|---|---|
| Compound according to example 1 | 5 mg |
| Lactose | 60 mg |
| Crystalline cellulose | 25 mg |
| K 90 Povidone | 5 mg |
| Pregelatinised starch | 3 mg |
| Colloidal silicon dioxide | 1 mg |
| Magnesium stearate | 1 mg |
| Total weight per tablet | 100 mg |

### Pharmacological data:

The binding of the inventively used sulphonamide derivatives of general formula (I) was determined as described above.

The binding results of some sulphonamide derivatives are given in the following table 1:

**Table 1:**

| **Compound according to example:** | **% Inhibition 10⁻⁶ M** | **Kᵢ (nM)** |
|---|---|---|
| 1 | 98.1 ± 4.0 | 0.28 |
| 3 | 96.6 ± 5.2 | 3.5 |
| 4 | 96.2 ± 0.6 | 9.3 |
| 5 | 101.2 ± 0.1 | 1.0 |
| 6 | 97.6 ± 1.8 | 8.7 |
| 7 | 103.0 ± 7.9 | 0.13 |
| 8 | 94.5 ± 7.0 | 0.76 |
| 9 | 96.8 ± 3.7 | 2.2 |
| 17 | 102.0 | 5.3 |

## Claims

1. Use of at least one sulphonamide derivative of general formula (I), 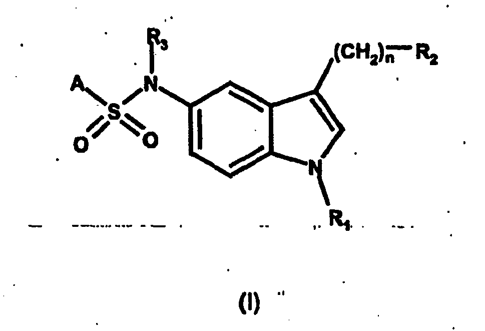 wherein
R¹ represents hydrogen, an optionally at least mono-substituted, linear or branched alkyl radical, an optionally at least mono-substituted phenyl radical or an optionally at least mono-substituted benzyl radical,
R² represents a -NR⁴R⁵ moiety,
R³ represents hydrogen or an optionally at least mono- substituted, linear or branched alkyl radical.
R⁴ and R⁵; identical or different, represent hydrogen or a linear or branched C₁-C₄-alkyl radical, or
R⁴ and R⁵ together with the bridging nitrogen atom form a moiety selected from the group consisting of 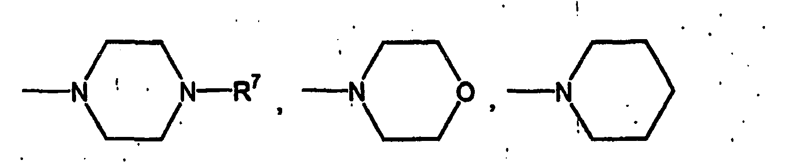 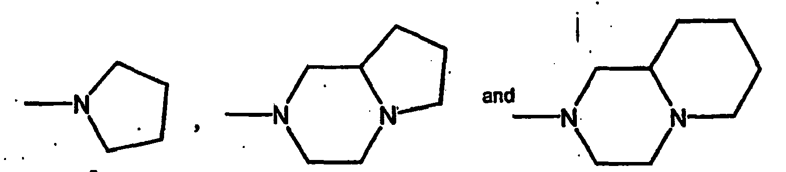 wherein R⁷ represents hydrogen, a linear or branched C₁-C₄-alkyl radical or a benzyl radical,
A represents an optionally at least mono-substituted mono- or polycyclic aromatic ringsystem, which may be bonded via an optionally at least mono-substituted alkylene-, an optionally at least mono-substituted alkenylene- or an optionally at least mono-substituted alkynylene group and/or may contain at least one heteroatom as a ring member in one or more of its rings,
n represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate,
for the manufacture of a medicament for the prophylaxis and/or treatment of a food ingestion disorder.

2. Use according to claim 1, **characterized in that** R¹ represents hydrogen, an optionally at least mono-substituted, linear or branched C₁₋₄-alkyl radical, an optionally at least mono-substituted phenyl radical or an optionally at least mono-substituted benzyl radical, preferably hydrogen, a linear or branched C₁₋₄-alkyl radical or a benzyl radical, more preferably hydrogen.

3. Use according to claim 1 or 2, **characterized in that** R³ represents hydrogen or an optionally at least mono-substituted, linear or branched C₁-C₄-alkyl radical, preferably hydrogen or a linear or branched C₁-C₄-alkyl radical, more preferably hydrogen or a C₁-C₂ alkyl radical.

4. Use according to any one of claims 1-3, **characterized in that** R⁴ an R⁵, identical or different, represent a linear or branched C₁-C₄-alkyl radical, or
R⁴ and R⁵ together with the bridging nitrogen atom form a moiety selected from the group consisting of 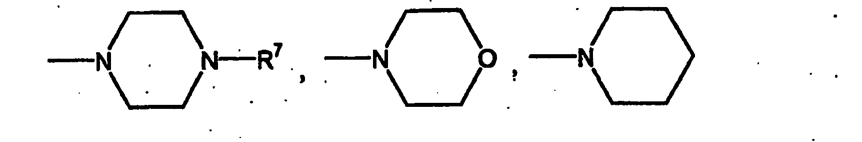 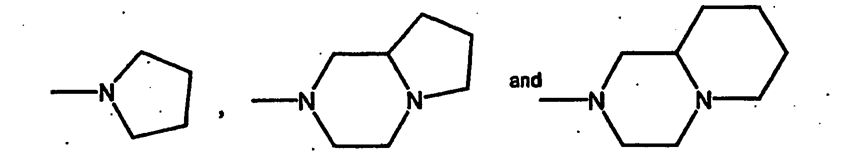 wherein R⁷ represents hydrogen or a C₁-C₂ alkyl radical.

5. Use according to any one of claims 1-4, **characterized in that** A represents an optionally at least mono-substituted mono- or bicyclic aromatic ringsystem, wherein the ring(s) is/are 5- or 6-membered, which may be bonded via a an optionally at least mono-substituted C₁-C₄-alkylene group, C₂-C₄-alkenylene or C₂-C₄-alkinylens group and/or may contain at least one heteroatom as a ring member, preferably an optionally at least mono-substituted mono- or bicyclic aromatic ringsystem, wherein the ring(s) is/are 5- or 6-membered and wherein one or both of the rings contain(s) at least one heteroatom, or a moiety selected from the group consisting of 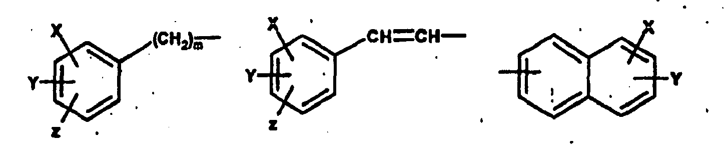 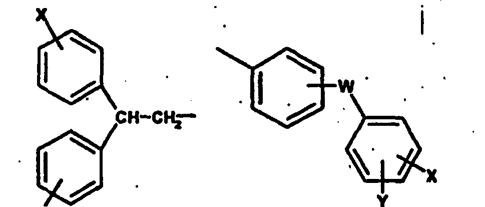 wherein X, Y, Z are each independently selected from the group consisting of hydrogen, fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR⁸R⁹-moiety, wherein R⁸ and R⁹, identical or different, represent hydrogen or linear or branched C₁-C₄-alkyl,
W represents a single chemical bond between the two rings, a CH₂-group, O, S or a NR¹⁰-moiety, wherein R¹⁰ is hydrogen or linear or branched C₁-C₄-alkyl and
m is 0, 1, 2, 3 or 4.

6. Use according to one or more of claims 1-5 of at least one sulphonamide derivative of general formula (I), 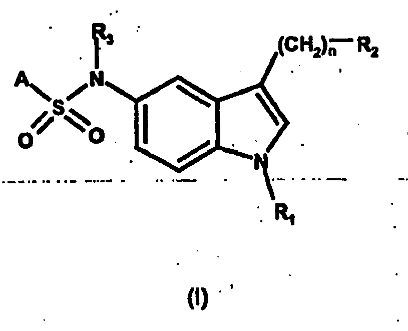 wherein
n represents 0, 1, 2, 3 or 4;
R¹ represents hydrogen,
R² represents a -NR⁴R⁵ moiety,
R³ represents hydrogen, a methyl group or an ethyl group,
R⁴ and R⁵, identical or different, represent a methyl group, an ethyl group, an n-propyl group, an iso-propyl group, an n-butyl group, an iso-butyl group, a sec-butyl group, or a tert.-butyl group, or
R⁴ and R⁵ together with the bridging nitrogen atom form a moiety selected from the group consisting 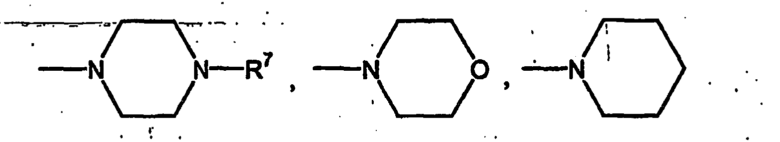 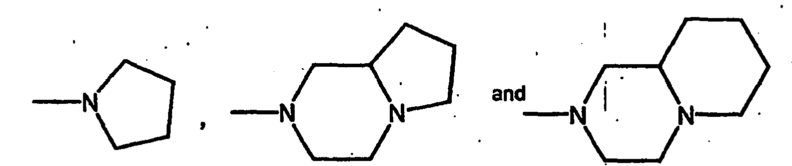 wherein R⁷ represents hydrogen, a methyl group or an ethyl group,
A represents a moiety selected from the group consisting of 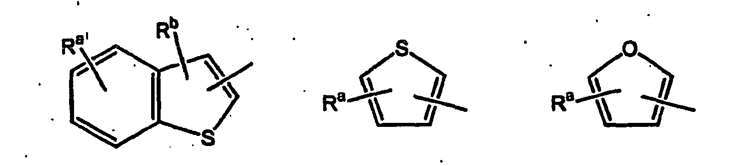 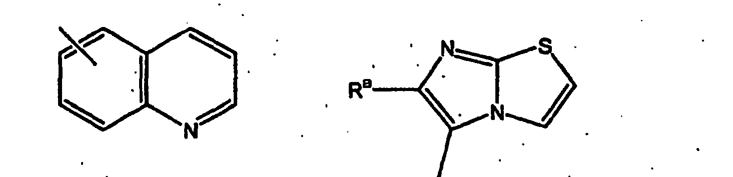 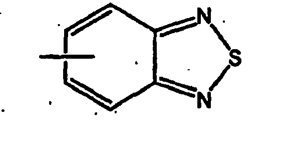 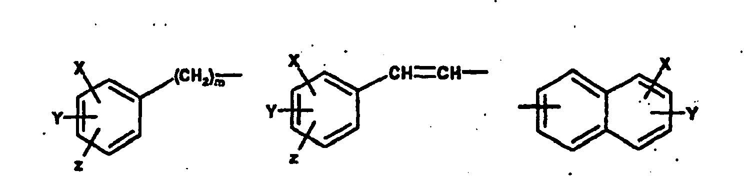 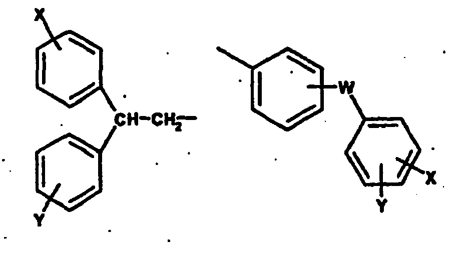 wherein
R^{A} and R^{B} are each independently selected from the group consisting of hydrogen, fluorine, chlorine, bromine, methyl, ethyl, pyridyl, thienyl and furyl,
X, Y, Z are each independently selected from the group consisting of hydrogen, fluorine, chlorine, bromine, methyl, ethyl, methoxy, ethoxy and -CF₃,
W represents a single chemical bond between the two rings, a CH₂-group, O, S or a NR¹⁰-moiety, wherein R¹⁰ is hydrogen, methyl or ethyl,
m is 0, 1, 2, 3 or 4.

7. Use according to any one of claims 1-6, **characterized in that** the compound is selected from the group consisting of:
[1] N-[3-(2-diethylaminoethyl)-1H-indol-5-yl]-5-chloro-3-methylbenzo[b]thiophene-2-sulphonamlde,
[2] N-[3-(2-diethylaminoethyl)-1H-indol-5-yl]naphthalene-1-sulphonamide,
[3] Hydrochloride N-[3-(2-diethylaminoethyl)-1*H*-indol-5-yl]naphthalene-1-sulphonamide,
[4] N-[3-(2-diethylaminoethyl)-1*H*-indol-5-yl]-3,5-dichlorobenzenesulphonamide,
[5] N-[3-(2-diethylaminoethyl)-1*H*-indol-5-yl]-4-phenylbenzenesulphonamide,
[6] N-[3-(2-diethylaminoethyl)-1*H*-indol-5-yl]-5-chlorothiophene-2-sulphonamide,
[7] N-[3-(2-dimethylaminoethyl)-1*H*-indol-5-yl]-5-chloro-3-methylbenzo[b]thiophene-2-sulphonamide,
[8] N-[3-(2-dimethylaminoethyl)-1*H*-indol-5-yl]naphthalene-1-sulphonamide,
[9] N-[3-(2-dimethylamino-ethyl)-1*H*-indol-5-yl]-6-chlorolmidazo[2,1-b]thiazol-5-sulphonamide,
[17] N-[3-(2-diethylaminoethyl)-1*H*-indol-5-yl]naphthalene-2-sulphonamide,
[19] N-[3-(4-methylpiperazin-1-yl)methyl-1*H*-indol-5-yl]-5-chloro-3-methylbenzo[b]thiophene-2-sulphonamide,
[20] N-[3-(2-dimethylaminoethyl)-1*H*-indol-6-yl]-5-(2-pyridil)thiophene-2-sulphonamide,
[21] N-[3-(2-dimethylaminoethyl)-1*H*-indol-5-yl]-2,1,3- benzothiadiazol-4-sulphonamide,
[22] N-[3-(2-dimethylaminoethyl)-1*H*-indol-5-yl]quinoline-8-sulphonamide,
[23] N-[3-(2-dimethylamlnoethyl)-1*H*-indol-5-yl]-5-chloronaphthalene-2-sulphonamide,
[24] N-[3-(2-dimethylaminoethyl)-1*H*-indol-5-yl]-4-phenoxybenzenesulphonamide,
[25] N-[3-(2-dimethytaminoethyl)-1*H*-indol-5-yl]-4-phenylbenzenesulphonamide,
[26] N-[3-(2-diethylaminoethyl)-1*H*-indol-5-yl]-N-ethyl-naphthalene-2-sulphonamide,
[27] N-{3-[2-(morpholin-4-yl)ethyl]-1H-indol-5-yl}-5-chlolo-3-methylbenzo[b]thiophene-2-sulphonamide,
[28] N-{3-[2-(morpholin-4-yl)ethyl]-1H-indol-5-yl}naphthalehe-1-sulphonamide,
[29] N-[3-(2-diethylaminoethyl)-1H-indol-5-yl]naphthalene-2-sulphonamide,
[30] N-[3-dimethylaminomethyl-1H-indol-5-yl]-5-chloro-3-methylbenzo[b]thiophene-2-sulphonamide,
[31] N-[3-(2-dipropylaminoethyl)-1H-indol-5-yl]naphthalene-1-sulphonamide,
[32] N-[3-(2-dipropylaminoethyl)-1H-indol-5-yl]-5-chloro-3-methylbenzo[b]thiophene-2-sulphonamide,
[33] N-[3-(2-dibutylaminoethyl)-1H-indol-5-yl]-5-chloro-3-methylbenzo[b]thiophene-2-sulphonamide,
[34] N-[3-(2-dibutylaminoethyl)-1H-indol-5-yl]naphthalene-1-sulphonamide,
[35] N-[3-(2-diethylaminoethyl)-1H-indol-5-yl]-5-chloronaphthalene-1-sulphonamide,
[36] N-[3-(2-diethylaminoethyl)-1H-indol-5-yl]-trans-β-styrenesulphonamide,
[37] N-[3-(4-methylpiperazin-1-yl)methyl-1*H*-indol-5-yl]-trans-β-styrenesulphonamide,
[39] N-[3-(2-diethylaminoethyl)-1*H*-indol-5-yl]-6-chloroimidazo[2,1-b]thiazol-5-sulphonamide,
[40] N-{3-[2-(morpholin-4-yl)ethyl]-1H-indol-5-yl}naphthalene-2-sulphonamide,
[41] N-[3-(4-methylpiperazin-1-yl)methyl-1*H*-indol-5-yl]-α-toluenesulphonamide,
[42] N-[3-(3-diethylaminopropyl)-1H-indol-5-yl]naphthalene-2-sulphonamide,
[43] N-[3-(3-diethylaminpropyl)-1H-indol-5-yl]-5-chloro-3-methylbenzo[b]thiophene-2-sulphonamide,
[44] N-{3-[2-(pyrrolidin-1-yl)ethyl]-1H-indol-5-yl}-5-chloro-3-methylbenzo[b]thiophene-2-sulphonamide,
[45] N-{3-[2-(pyrrolidin-1-yl)ethyl]-1H-indol-5-yl}naphthalene-1-sulphonamide,
[46] N-{3-[2-(pyrrolidin-1-yl)ethyl]-1H-indol-5-yl}naphthalene-2-sulphonamide,
[47] N-[3-(2-dipropylaminoethyl)-1H-indol-5-yl]naphthalene-2-sulphonamide,
[48] N-[3-(2-dimethylaminoethyl)-1H-indol-5-yl]-5-chloronaphthalene-1-sulphonamide,
[49] N-[3-(2-dimethylaminoethyl)-1H-indol-5-yl]naphthalene-2-sulphonamide,
[50] N-{3-[2-(morpholin-4-yl)ethyl]-1H-indol-5-yl}quinoline-8-sulphonamide;
[51] N-{3-[2-(morpholin-4-yl)ethyl]-1H-indol-5-yl}-4-phenylbenzenesulphonamide.

8. Use according to any one of claims 1-7 for the regulation of appetite.

9. Use according to any one of claims 1-7 for the reduction, increase or maintenance of body weight.

10. Use according to any one of claims 1-7 for the prophylaxis and/or treatment of obesity.

11. Use according to any one of claims 1-7 for the prophylaxis and/or treatment of bulimia.

12. Use according to any one of claims 1-7 for the prophylaxis and/or treatment of anorexia.

13. Use according to any one of claims 1-7 for the prophylaxis and/or treatment of cachexia.

14. Use according to any one of claims 1-7 for the prophylaxis and/or treatment of type II diabetes.

## Patentansprüche

1. Verwendung von wenigstens einem Sulfonamidderivat der allgemeinen Formel (I) 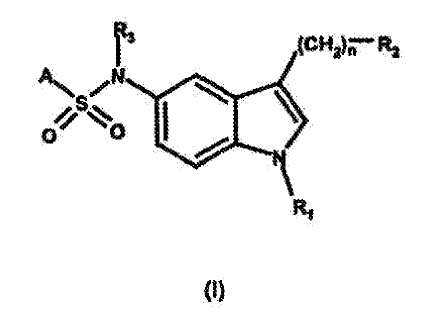 worin
R¹ Wasserstoff, einen gegebenenfalls wenigstens monosubstituierten, linearen oder verzweigten Alkylrest, einen gegebenenfalls wenigstens monosubstituierten Phenylrest oder einen gegebenenfalls wenigstens monosubstituierten Benzylrest bedeutet,
R² eine -NR⁴R⁵ Einheit bedeutet,
R³ Wasserstoff oder einen gegebenenfalls wenigstens monosubstituierten, linearen oder verzweigten Alkylrest bedeutet,
R⁴ und R⁵, identisch oder verschieden, Wasserstoff oder einen linearen oder verzweigten C₁-C₄-Alkylrest bedeuten, oder
R⁴ und R⁵ zusammen mit dem verbrückenden Stickstoffatom eine Einheit bilden, die ausgewählt ist aus der Gruppe bestehend aus 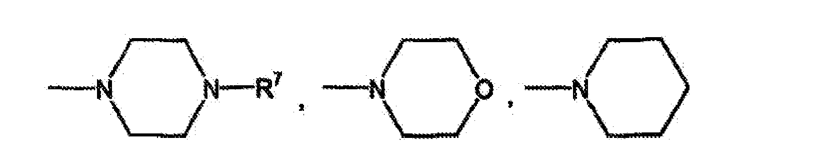 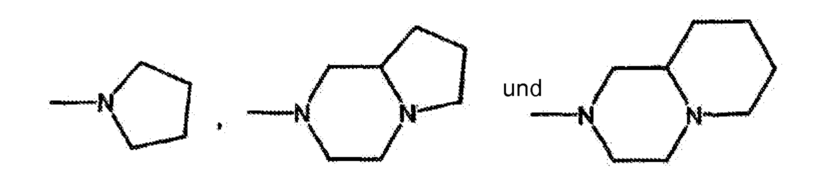 worin R⁷ Wasserstoff, einen linearen oder verzweigten C₁-C₄-Alkylrest oder einen Benzylrest bedeutet,
A ein gegebenenfalls wenigstens monosubstituiertes mono- oder polycyclisches aromatisches Ringsystem bedeutet, welches über eine gegebenenfalls wenigstens monosubstituierte Alkylengruppe, eine gegebenenfalls wenigstens monosubstituierte Alkenylengruppe oder eine gegebenenfalls wenigstens monosubstituierte Alkinylengruppe gebunden sein kann und/oder wenigstens ein Heteroatom als ein Ringglied in einem oder mehreren seiner Ringe enthalten kann,
n 0, 1, 2, 3 oder 4 bedeutet;
gegebenenfalls in Form von einem seiner Stereoisomere, vorzugsweise Enantiomere oder Diastereomere, seines Racemats oder in Form einer Mischung von wenigstens zwei von seinen Stereoisomeren, vorzugsweise Enantiomeren oder Diastereomeren, in einem beliebigen Mischungsverhältnis, oder einem entsprechenden physiologisch verträglichen Salz oder einem entsprechenden Solvat,
für die Herstellung eines Medikaments für die Prophylaxe und/oder Behandlung einer Störung der Nahrungsmittelaufnahme.

2. Verwendung nach Anspruch 1, **dadurch gekennzeichnet, dass** R¹ Wasserstoff, einen gegebenenfalls wenigstens monosubstituierten linearen oder verzweigten C₁₋₄-Alkylrest, einen gegebenenfalls wenigstens monosubstituierten Phenylrest oder einen gegebenenfalls wenigstens monosubstituierten Benzylrest, vorzugsweise Wasserstoff, einen linearen oder verzweigten C₁₋₄-Alkylrest oder einen Benzylrest, mehr bevorzugt Wasserstoff bedeutet.

3. Verwendung nach Anspruch 1 oder 2, **dadurch gekennzeichnet, dass** R³ Wasserstoff oder einen gegebenenfalls wenigstens monosubstituierten linearen oder verzweigten C₁-C₄-Alkylrest, vorzugsweise Wasserstoff, oder einen linearen oder verzweigten C₁-C₄-Alkylrest, mehr bevorzugt Wasserstoff oder einen C₁-C₂-Alkylrest bedeutet.

4. Verwendung nach einem der Ansprüche 1-3, **dadurch gekennzeichnet, dass** R⁴ und R⁵, identisch oder verschieden, einen linearen oder verzweigten C₁-C₄-Alkylrest bedeuten oder
R⁴ und R⁵ zusammen mit dem verbrückenden Stickstoffatom eine Einheit bilden, die ausgewählt ist aus der Gruppe bestehend aus 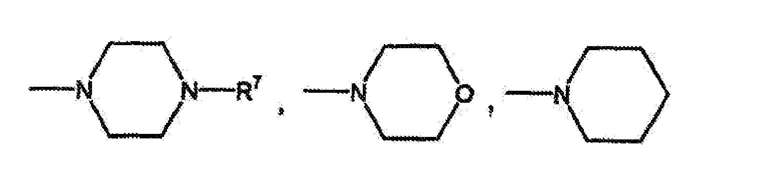 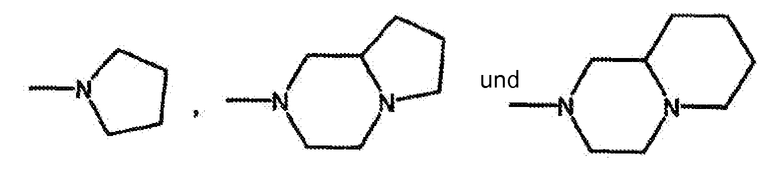 worin R⁷ Wasserstoff oder einen C₁-C₂-Alkylrest bedeutet.

5. Verwendung nach einem der Ansprüche 1-4, **dadurch gekennzeichnet, dass** A ein gegebenenfalls wenigstens monosubstituiertes mono- oder bicyclisches aromatisches Ringsystem bedeutet, wobei der Ring bzw. die Ringe 5- oder 6-gliedrig ist/sind, welches über eine gegebenenfalls wenigstens monosubstituierte C₁-C₄-Alkylengruppe, C₂-C₄-Alkenylengruppe oder C₂-C₄-Alkinylengruppe gebunden sein kann und/oder wenigstens ein Heteroatom als ein Ringglied enthalten kann, vorzugsweise ein gegebenenfalls wenigstens monosubstituiertes mono- oder bicyclisches aromatisches Ringsystem, wobei der Ring bzw. die Ringe 5- oder 6-gliedrig ist/sind und wobei einer oder beide der Ringe wenigstens ein Heteroatom enthält (enthalten), oder eine Einheit, ausgewählt aus der Gruppe bestehend aus 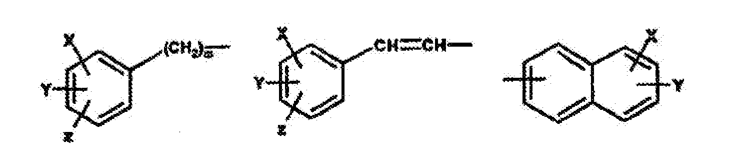 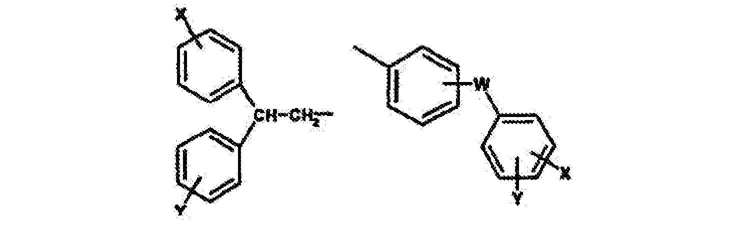 worin X, Y, Z jeweils unabhängig voneinander ausgewählt sind aus der Gruppe bestehend aus Wasserstoff, Fluor, Chlor, Brom, linearem oder verzweigtem C₁-C₄-Alkyl, linearem oder verzweigtem C₁-C₄-Alkoxy, linearem oder verzweigtem C₁-C₄-Alkylthio, einer Trifluormethyleinheit, einer Cyanoeinheit und einer NR⁸R⁹-Einheit, worin R⁸ und R⁹, identisch oder verschieden, Wasserstoff oder lineares oder verzweigtes C₁-C₄-Alkyl bedeuten,
W eine chemische Einfachbindung zwischen den zwei Ringen, eine CH₂-Gruppe, O, S oder eine NR¹⁰-Einheit bedeutet, wobei R¹⁰ Wasserstoff oder lineares oder verzweigtes C₁-C₄-Alkyl ist und
m 0, 1, 2, 3 oder 4 ist.

6. Verwendung nach einem oder mehreren der Ansprüche 1-5 von wenigstens einem Sulfonamidderivat der allgemeinen Formel (I), 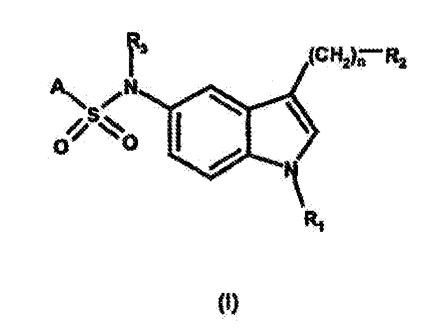 worin
n 0, 1, 2, 3 oder 4 bedeutet;
R¹ Wasserstoff bedeutet,
R² eine -NR⁴R⁵ Einheit bedeutet,
R³ Wasserstoff, eine Methylgruppe oder eine Ethylgruppe bedeutet,
R⁴ und R⁵, identisch oder verschieden, eine Methylgruppe, eine Ethylgruppe, eine n-Propylgruppe, eine Isopropylgruppe, eine n-Butylgruppe, eine Isobutylgruppe, eine sec-Butylgruppe oder eine tert-Butylgruppe bedeuten, oder
R⁴ und R⁵ zusammen mit dem verbrückenden Stickstoffatom eine Einheit bilden, die ausgewählt ist aus der Gruppe bestehend aus 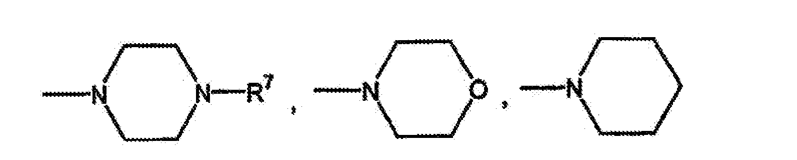 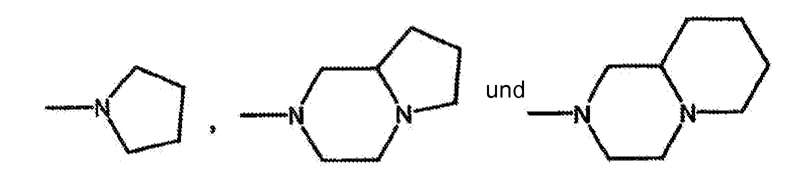 worin R⁷ Wasserstoff, eine Methylgruppe oder eine Ethylgruppe bedeutet,
A eine Einheit bedeutet, die ausgewählt ist aus der Gruppe bestehend aus 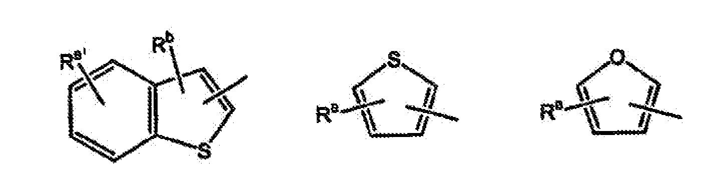 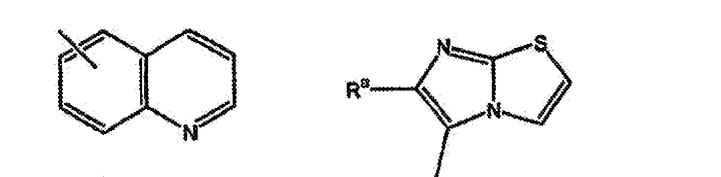 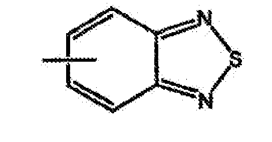 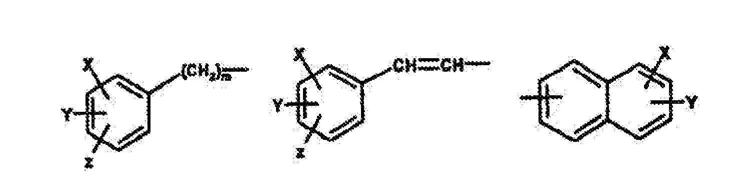 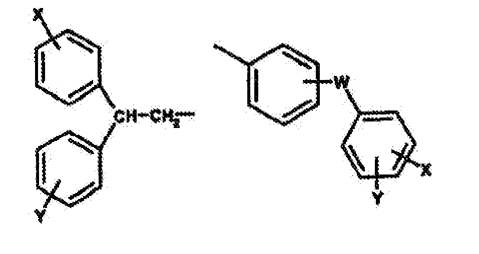 worin
R^{A} und R^{B} jeweils unabhängig voneinander ausgewählt sind aus der Gruppe bestehend aus Wasserstoff, Fluor, Chlor, Brom, Methyl, Ethyl, Pyridyl, Thienyl und Furyl,
X, Y, Z jeweils unabhängig voneinander ausgewählt sind aus der Gruppe bestehend aus Wasserstoff, Fluor, Chlor, Brom, Methyl, Ethyl, Methoxy, Ethoxy und -CF₃,
W eine chemische Einfachbindung zwischen den zwei Ringen, eine CH₂-Gruppe, O, S oder eine NR¹⁰-Einheit bedeutet, worin R¹⁰ Wasserstoff, Methyl oder Ethyl ist,
m 0, 1, 2, 3 oder 4 ist.

7. Verwendung nach einem der Ansprüche 1-6, **dadurch gekennzeichnet, dass** die Verbindung ausgewählt ist aus der Gruppe bestehend aus:
[1] N-[3-(2-Diethylaminoethyl)-1H-indol-5-yl]-5-chlor-3-methylbenzo[b]thiophen-2-sulfonamid,
[2] N-[3-(2-Diethylaminoethyl)-1H-indol-5-yl]naphthalin-1-sulfonamid,
[3] N-[3-(2-Diethylaminoethyl)-1H-indol-5-yl]naphthalin-1-sulfonamid-Hydrochlorid,
[4] N-[3-(2-Diethylaminoethyl)-1H-indol-5-yl]-3,5-dichlorbenzolsulfonamid,
[5] N-[3-(2-Diethylaminoethyl)-1H-indol-5-yl]-4-phenylbenzolsulfonamid,
[6] N-[3-(2-Diethylaminoethyl)-1H-indol-5-yl]-5-chlorthiophen-2-sulfonamid,
[7] N-[3-(2-Dimethylaminoethyl)-1H-indol-5-yl]-5-chlor-3-methylbenzo[b]thiophen-2-sulfonamid,
[8] N-[3-(2-Dimethylaminoethyl)-1H-indol-5-yl]naphthalin-1-sulfonamid,
[9] N-[3-(2-Dimethylamino-ethyl)-1H-indol-5-yl]-6-chlorimidazo[2,1-b]thiazol-5-sulfonamid,
[17] N-[3-(2-Diethylaminoethyl)-1 H-indol-5-yl]naphthalin-2-sulfonamid,
[19] N-[3-(4-Methylpiperazin-1-yl)methyl-1 H-indol-5-yl]-5-chlor-3-methylbenzo[b]thiophen-2-sulfonamid,
[20] N-[3-(2-Dimethylaminoethyl)-1H-indol-5-yl]-5-(2-pyridyl)thiophen-2-sulfonamid,
[21] N-[3-(2-Dimethylaminoethyl)-1H-indol-5-yl]-2,1,3-benzothiadiazol-4-sulfonamid,
[22] N-[3-(2-Dimethylaminoethyl)-1H-indol-5-yl]chinolin-8-sulfonamid,
[23] N-[3-(2-Dimethylaminoethyl)-1H-indol-5-yl]-5-chlornaphthalin-2-sulfonamid,
[24] N-[3-(2-Dimethylaminoethyl)-1H-indol-5-yl]-4-phenoxybenzolsulfonamid,
[25] N-[3-(2-Dimethylaminoethyl)-1H-indol-5-yl]-4-phenylbenzolsulfonamid,
[26] N-[3-(2-Diethylaminoethyl)-1H-indol-5-yl]-N-ethyl-naphthalin-2-sulfonamid,
[27] N-{3-[2-(Morpholin-4-yl)ethyl]-1H-indol-5-yl}-5-chlor-3-methylbenzo[b]thiophen-2-sulfonamid,
[28] N-{3-[2-(Morpholin-4-yl)ethyl]-1H-indol-5-yl}naphthalin-1-sulfonamid,
[29] N-[3-(2-Diethylaminoethyl)-1H-indol-5-yl]naphthalin-2-sulfonamid,
[30] N-[3-Dimethylaminomethyl-1H-indol-5-yl]-5-chlor-3-methylbenzo[b]thiophen-2-sulfonamid,
[31] N-[3-(2-Dipropylaminoethyl)-1H-indol-5-yl]naphthalin-1-sulfonamid,
[32] N-[3-(2-Dipropylaminoethyl)-1H-indol-5-yl]-5-chlor-3-methylbenzo[b]thiophen-2-sulfonamid,
[33] N-[3-(2-Dibutylaminoethyl)-1H-indol-5-yl]-5-chlor-3-methylbenzo[b]thiophen-2-sulfonamid,
[34] N-[3-(2-Dibutylaminoethyl)-1H-indol-5-yl]naphthalin-1-sulfonamid,
[35] N-[3-(2-Diethylaminoethyl)-1H-indol-5-yl]-5-chlornaphthalin-1-sulfonamid,
[36] N-[3-(2-Diethylaminoethyl)-1H-indol-5-yl]-trans-β-styrolsulfonamid,
[37] N-[3-(4-Methylpiperazin-1-yl)methyl-1H-indol-5-yl]-trans-β-styrolsulfonamid,
[39] N-[3-(2-Diethylaminoethyl)-1 H-indol-5-yl]-6-chlorimidazo[2,1-b]thiazol-5-sulfonamid,
[40] N-{3-[2-(Morpholin-4-yl)ethyl]-1H-indol-5-yl}naphthalin-2-sulfonamid,
[41] N-[3-(4-Methylpiperazin-1-yl)methyl-1H-indol-5-yl]-α-toluolsulfonamid,
[42] N-[3-(3-Diethylaminopropyl)-1H-indol-5-yl]naphthalin-2-sulfonamid,
[43] N-[3-(3-Diethylaminopropyl)-1H-indol-5-yl]-5-chlor-3-methylbenzo[b]thiophen-2-sulfonamid,
[44] N-{3-[2-(Pyrrolidin-1-yl)ethyl]-1H-indol-5-yl}-5-chlor-3-methylbenzo[b]thiophen-2-sulfonamid,
[45] N-{3-[2-(Pyrrolidin-1-yl)ethyl]-1H-indol-5-yl}naphthalin-1-sulfonamid,
[46] N-{3-[2-(Pyrrolidin-1-yl)ethyl]-1H-indol-5-yl}naphthalin-2-sulfonamid,
[47] N-[3-(2-Dipropylaminoethyl)-1H-indol-5-yl]naphthalin-2-sulfonamid,
[48] N-[3-(2-Dimethylaminoethyl)-1H-indol-5-yl]-5-chlornaphthalin-1-sulfonamid,
[49] N-[3-(2-Dimethylaminoethyl)-1H-indol-5-yl]naphthalin-2-sulfonamid,
[50] N-{3-[2-(Morpholin-4-yl)ethyl]-1H-indol-5-yl}chinolin-8-sulfonamid,
[51] N-{3-[2-(Morpholin-4-yl)ethyl]-1H-indol-5-yl}-4-phenylbenzolsulfonamid,

8. Verwendung nach einem der Ansprüche 1-7 zur Regelung des Appetits.

9. Verwendung nach einem der Ansprüche 1-7 zur Verringerung, Erhöhung oder Aufrechterhaltung des Körpergewichts.

10. Verwendung nach einem der Ansprüche 1-7 für die Prophylaxe und/oder Behandlung von Fettleibigkeit.

11. Verwendung nach einem der Ansprüche 1-7 für die Prophylaxe und/oder Behandlung von Bulimie.

12. Verwendung nach einem der Ansprüche 1-7 für die Prophylaxe und/oder Behandlung von Anorexie.

13. Verwendung nach einem der Ansprüche 1-7 für die Prophylaxe und/oder Behandlung von Kachexie.

14. Verwendung nach einem der Ansprüche 1-7 für die Prophylaxe und/oder Behandlung von Typ II Diabetes.

## Revendications

1. Utilisation d'au moins un dérivé sulfamide de formule générale (I) : 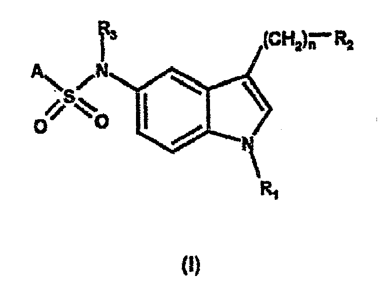 dans laquelle
R¹ représente l'hydrogène, un radical alkyle linéaire ou ramifié éventuellement au moins monosubstitué, un radical phényle éventuellement au moins monosubstitué ou un radical benzyle éventuellement au moins monosubstitué,
R² représente un fragment -NR⁴R⁵,
R³ représente l'hydrogène ou un radical alkyle linéaire ou ramifié éventuellement au moins monosubstitué,
R⁴ et R⁵, identiques ou différents, représentent l'hydrogène ou un radical alkyle en C₁-C₄ linéaire ou ramifié, ou
R⁴ et R⁵, conjointement avec l'atome d'azote pontant, forment un fragment choisi dans le groupe constitué par : 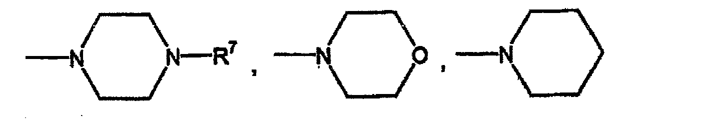 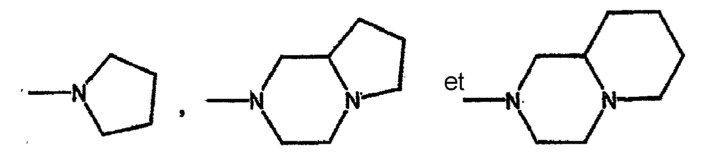 dans lequel R⁷ représente l'hydrogène, un radical alkyle en C₁-C₄ linéaire ou ramifié ou un radical benzyle,
A représente un système de noyau(x) aromatique(s) monocyclique(s) ou polycyclique(s) éventuellement au moins monosubstitué qui peut être fixé par un groupe alkylène éventuellement au moins monosubstitué, un groupe alcénylène éventuellement au moins monosubstitué ou un groupe alcynylène éventuellement au moins monosubstitué et/ou qui peut contenir au moins un hétéroatome en tant que noyau dans un ou plusieurs de ses noyaux,
n représente 0, 1, 2, 3 ou 4 ;
éventuellement sous la forme d'un de ses stéréoisomères, de préférence les énantiomères ou les diastéréomères, de son racémate ou sous la forme d'un mélange d'au moins deux de ses stéréoisomères, de préférence les énantiomères ou les diastéréomères, selon n'importe quel rapport de mélange ou un sel correspondant acceptable physiologiquement ou bien un solvate correspondant,
pour la fabrication d'un médicament pour la prophylaxie et/ou le traitement d'un trouble d'ingestion des aliments.

2. Utilisation selon la revendication 1,
**caractérisée en ce que**
R¹ représente l'hydrogène, un radical alkyle en C₁-C₄ linéaire ou ramifié éventuellement au moins monosubstitué, un radical phényle éventuellement au moins monosubstitué ou un radical benzyle éventuellement au moins monosubstitué, de préférence l'hydrogène, un radical alkyle en C₁-C₄ linéaire ou ramifié ou un radical benzyle, de manière davantage préférée, l'hydrogène.

3. Utilisation selon la revendication 1 ou 2,
**caractérisée en ce que**
R³ représente l'hydrogène ou un radical alkyle en C₁-C₄ linéaire ou ramifié éventuellement au moins monosubstitué, de préférence l'hydrogène ou un radical alkyle en C₁-C₄ linéaire ou ramifié, de manière davantage préférée, l'hydrogène ou un radical alkyle en C₁-C_{2.}

4. Utilisation selon l'une quelconque des revendications 1 à 3,
**caractérisée en ce que**
R⁴ et R⁵, identiques ou différents, représentent un radical alkyle en C₁-C₄ linéaire ou ramifié, ou
R⁴ et R⁵, conjointement avec l'atome d'azote pontant, forment un fragment choisi dans le groupe constitué par 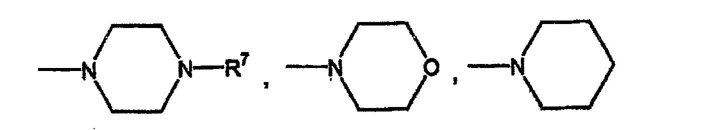 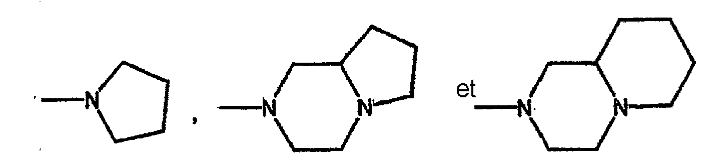 dans lequel R⁷ représente l'hydrogène ou un radical alkyle en C₁-C₂.

5. Utilisation selon l'une quelconque des revendications 1 à 4,
**caractérisée en ce que**
A représente un système de noyau(x) aromatique(s) monocyclique(s) ou bicyclique(s) éventuellement au moins monosubstitué, dans lequel le(s) noyau(x) a/ont 5 ou 6 chaînons, qui peut fixé par un groupe alkylène en C₁-C₄, un groupe alcénylène en C₂-C₄ ou un groupe alcynylène en C₂-C₄ éventuellement au moins monosubstitué et/ou qui peut contenir au moins un hétéroatome en tant que noyau, de préférence un système de noyau(x) aromatique(s) monocyclique(s) ou bicyclique(s) éventuellement au moins monosubstitué, dans lequel le(s) noyau(x) a/ont 5 ou 6 chaînons et dans lequel un ou plusieurs des noyaux contien(nen)t au moins un hétéroatome ou un fragment choisi dans le groupe constitué par : 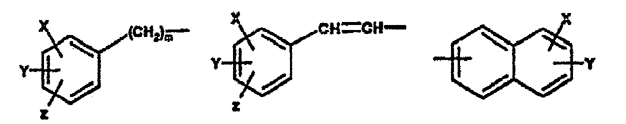 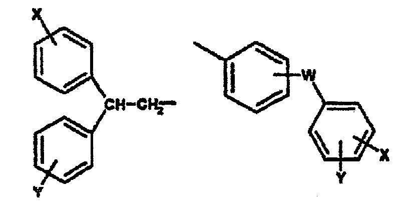 dans lequel X, Y et Z sont chacun choisis indépendamment dans le groupe constitué par l'hydrogène, le fluor, le chlore, le brome, un radical alkyle en C₁-C₄ linéaire ou ramifié, un radical alcoxy en C₁-C₄ linéaire ou ramifié, un radical alkylthio en C₁-C₄ linéaire ou ramifié, un fragment trifluorométhyle, un fragment cyano et un fragment NR⁸R⁹, dans lequel R⁸ et R⁹, identiques ou différents, représentent l'hydrogène ou un radical alkyle en C₁-C₄ linéaire ou ramifié,
W représente une liaison chimique simple entre les deux noyaux, un groupe CH₂, O, S ou un fragment NR¹⁰, dans lequel R¹⁰ est l'hydrogène ou un radical alkyle C₁-C₄ linéaire ou ramifié et
m vaut 0, 1, 2, 3 ou 4.

6. Utilisation selon une ou plusieurs des revendications 1 à 5 d'au moins un dérivé sulfamide de formule générale (I) : 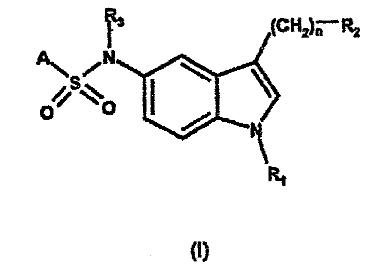 dans laquelle
n représente 0, 1, 2, 3 ou 4 ;
R¹ représente l'hydrogène,
R² représente un fragment -NR⁴R⁵,
R³ représente l'hydrogène, un groupe méthyle ou un groupe éthyle,
R⁴ et R⁵, identiques ou différents, représentent un groupe méthyle, un groupe éthyle, un groupe n-propyle, un groupe isopropyle, un groupe n-butyle, un groupe isobutyle, un groupe sec-butyle ou un groupe tert-butyle, ou
R⁴ et R⁵, conjointement avec l'atome d'azote pontant, forment un fragment choisi dans le groupe constitué par : 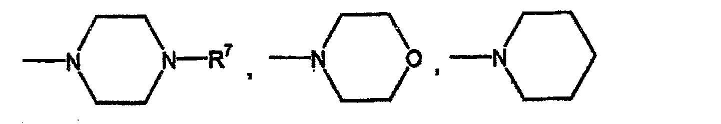 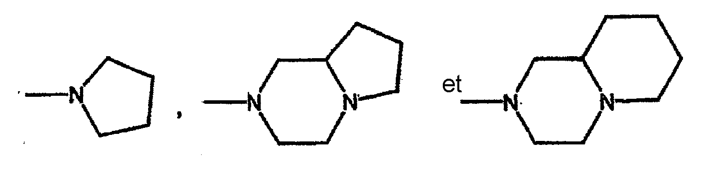 dans lequel R⁷ représente l'hydrogène, un groupe méthyle ou un groupe éthyle,
A représente un fragment choisi dans le groupe constitué par : 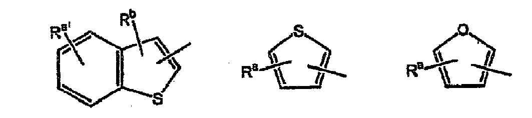 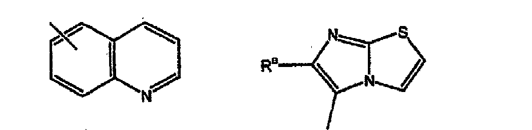 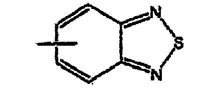 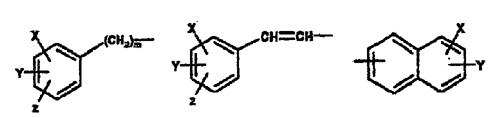 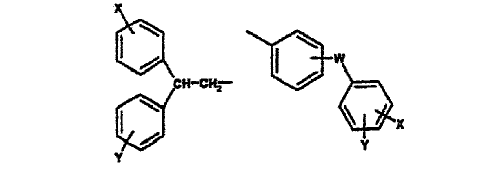 dans lequel
R^{A} et R^{B} sont choisis chacun indépendamment dans le groupe constitué par l'hydrogène, le fluor, le chlore, le brome, le méthyle, l'éthyle, le pyridyle, le thiényle et le furyle,
X ,Y, Z sont choisis chacun indépendamment dans le groupe constitué par l'hydrogène, le fluor, le chlore, le brome, le méthyle, l'éthyle, le méthoxy, l'éthoxy et -CF₃,
W représente une liaison chimique unique entre les deux cycles, un groupe CH₂, O, S ou un fragment NR¹⁰, dans lequel R¹⁰ est l'hydrogène, le méthyle ou l'éthyle,
m vaut 0, 1, 2, 3 ou 4.

7. Utilisation selon l'une quelconque des revendications 1 à 6,
**caractérisée en ce que**
le composé est choisi dans le groupe constitué par :
[1] le N-[3-(2-diéthylaminoéthyl)-1H-indol-5-yl]-5-chloro-3-méthylbenzo[b]thiophène-2-sulfamide,
[2] le N-[3-(2-diéthylaminoéthyl)-1H-indol-5-yl)naphtalène-1-sulfamide,
[3] le chlorhydrate de N-[3-(2-diéthylaminoéthyl)-1H-indol-5-yl]naphtalène-1-sulfamide,
[4] le N-[3-(2-diéthylaminoéthyl)-1H-indol-5-yl]-3,5-dichlorobenzènesulfamide,
[5] le N-[3-(2-diéthylaminoéthyl)-1H-indol-5-yl]-4-phénylbenzènesulfamide,
[6] le N-[3-(2-diéthylaminoéthyl)-1H-indol-5-yl]-5-chlorothiophène-2-sulfamide,
[7] le N-[3-(2-diméthylaminoéthyl)-1H-indol-5-yl]-5-chloro-3-méthylbenzo[b]thiophène-2-sulfamide,
[8] le N-[3-(2-diméthylaminoéthyl)-1H-indol-5-yl]naphtalène-1-sulfamide,
[9] le N-[3-(2-diméthylaminoéthyl)-1H-indol-5-yl]-6-chloroimidazo[2,1-b]thiazol-5-sulfamide,
[17] le N-[3-(2-diéthylaminoéthyl)-1H-indol-5-yl]naphtalène-2-sulfamide,
[19] le N-[3-(4-méthylpipérazin-1-yl)méthyl-1H-indol-5-yl]-5-chloro-3-méthylbenzo[b]thiophène-2-sulfamide,
[20] le N-[3-(2-diméthylaminoéthyl)-1H-indol-5-yl]-5-(2-pyridyl)thiophène-2-sulfamide,
[21] le N-[3-(2-diméthylaminoéthyl)-1H-indol-5-yl]-2,1,3-benzothiadiazol-4-sulfamide,
[22] le N-[3-(2-diméthylaminoéthyl)-1H-indol-5-yl]quinoline-8-sulfamide,
[23] le N-[3-(2-diméthylaminoéthyl)-1H-indol-5-yl]-5-chloronaphtalène-2-sulfamide,
[24] le N-[3-(2-diméthylaminoéthyl)-1H-indol-5-yl]-4-phénoxybenzènesulfamide,
[25] le N-[3-(2-diméthylaminoéthyl)-1H-indol-5-yl]-4-phénylbenzènesulfamide,
[26] le N-[3-(2-diéthylaminoéthyl)-1H-indol-5-yl]-N-éthyl-naphtalène-2-sulfamide,
[27] le N-{3-[2-(morpholin-4-yl)éthyl]-1H-indol-5-yl}-5-chloro-3-méthylbenzo[b]thiophène-2-sulfamide,
[28] le N-{3-[2-(morpholin-4-yl)éthyl]-1H-indol-5-yl}naphtalène-1-sulfamide,
[29] le N-[3-(2-diéthylaminoéthyl)-1H-indol-5-yl]naphtalène-2-sulfamide,
[30] le N-[3-diméthylaminométhyl-1H-indol-5-yl]-5-chloro-3-méthylbenzo[b]thiophène-2-sulfamide,
[31] le N-[3-(2-dipropylaminoéthyl)-1H-indol-5-yl]naphtalène-1-sulfamide,
[32] le N-[3-(2-dipropylaminoéthyl)-1H-indol-5-yl]-5-chloro-3-méthylbenzo[b]thiophène-2-sulfamide,
[33] le N-[3-(2-dibutylaminoéthyl)-1H-indol-5-yl]-5-chloro-3-méthylbenzo[b]thiophène-2-sulfamide,
[34] le N-[3-(2-dibutylaminoéthyl)-1H-indol-5-yl]naphtalène-1-sulfamide,
[35] le N-[3-(2-diéthylaminoéthyl)-1H-indol-5-yl]-5-chloronaphtalène-1-sulfamide,
[36] le N-[3-(2-diéthylaminoéthyl)-1H-indol-5-yl]-trans-β-styrènesulfamide,
[37] le N-[3-(4-méthylpipérazin-1-yl)méthyl-1H-indol-5-yl]-trans-β-styrènesulfamide,
[39] le N-[3-(2-diéthylaminoéthyl)-1H-indol-5-yl]-6-chloroimidazo[2,1-b]thiazol-5-sulfamide,
[40] le N-{3-[2-(morpholin-4-yl)éthyl]-1H-indol-5-yl}-naphtalène-2-sulfamide,
[41] le N-[3-(4-méthylpipérazin-1-yl)méthyl-1H-indol-5-yl]-α-toluènesulfamide,
[42] le N-[3-(3-diéthylaminopropyl)-1H-indol-5-yl]naphtalène-2-sulfamide,
[43] le N-[3-(3-diéthylaminopropyl)-1H-indol-5-yl]-5-chloro-3-méthylbenzo[b]thiophène-2-sulfamide,
[44] le N-{3-[2-(pyrrolidin-l-yl)éthyl]-1H-indol-5-yl}-5-chloro-3-méthylbenzo[b]thiophène-2-sulfamide,
[45] le N-{3-[2-(pyrrolidin-l-yl)éthyl]-1H-indol-5-yl}naphtalène-1-sulfamide,
[46] le N-{3-[2-(pyrrolidin-1-yl)éthyl]-1H-indol-5-yl}naphtalène-2-sulfamide,
[47] le N-[3-(2-dipropylaminoéthyl)-1H-indol-5-yl]naphtalène-2-sulfamide,
[48] le N-[3-(2-diméthylaminoéthyl)-1H-indol-5-yl]-5-chloronaphtalène-1-sulfamide,
[49] le N-[3-(2-diméthylaminoéthyl)-1H-indol-5-yl]naphtalène-2-sulfamide,
[50] le N-{3-[2-(morpholin-4-yl)éthyl]-1H-indol-5-yl}quinoline-8-sulfamide,
[51] le N-{3-[2-(morpholin-4-yl)éthyl]-1H-indol-5-yl}-4-phénylbenzènesulfamide.

8. Utilisation selon l'une quelconque des revendications 1 à 7 pour réguler l'appétit.

9. Utilisation selon l'une quelconque des revendications 1 à 7 pour réduire, augmenter ou maintenir le poids corporel.

10. Utilisation selon l'une quelconque des revendications 1 à 7 pour la prophylaxie et/ou le traitement de l'obésité.

11. Utilisation selon l'une quelconque des revendications 1 à 7 pour la prophylaxie et/ou le traitement de la boulimie.

12. Utilisation selon l'une quelconque des revendications 1 à 7 pour la prophylaxie et/ou le traitement de l'anorexie.

13. Utilisation selon l'une quelconque des revendications 1 à 7 pour la prophylaxie et/ou le traitement de la cachexie.

14. Utilisation selon l'une quelconque des revendications 1 à 7 pour la prophylaxie et/ou le traitement du diabète de type II.
